Europäisches Patentamt

European Patent Office

Office européen des brevets

(19)

(11) Publication number: **0 292 009**

**A1**

(12) **EUROPEAN PATENT APPLICATION**

(21) Application number: 88108148.3

(22) Date of filing: 20.05.88

(51) Int. Cl.⁴: **C12N 9/50 , C12N 15/00 , C07H 21/04 , A61K 37/54 , C12N 5/00**

The microorganism(s) has (have) been deposited with American Type Culture Collection under number(s) ATCC 53347, 53445, 20728.

(30) Priority: 22.05.87 US 53411

(43) Date of publication of application:
23.11.88 Bulletin 88/47

(84) Designated Contracting States:
AT BE CH DE ES FR GB GR IT LI LU NL SE

(71) Applicant: ZYMOGENETICS INC.
4225 Roosevelt Way, N.E.
Seattle Washington 98105(US)

(72) Inventor: Mulvihill, Eileen R.
4016 Francis Avenue North
Seattle Washington 98103(US)
Inventor: Kumar, Anur Ashok
4509 Fremont Avenue North No. 4
Seattle Washington 98103(US)
Inventor: Foster, Donald C.
3002 Northeast 181st Street
Seattle Washington 98155(US)
Inventor: Rao, Donald D.
3716 Northeast 75th, No. 105
Seattle Washington 98155(US)
Inventor: O'Hara, Patrick J.
4318 Winslow Place North
Seattle Washington 98103(US)
Inventor: Insley, Margaret Y.
16860 Northeast 150th
Woodinville Washington 98072(US)

(74) Representative: Brown, John David et al
FORRESTER & BOEHMERT
Widenmayerstrasse 4/I
D-8000 München 22(DE)

(54) **Fibrinolytic proteins.**

(57) Tissue-type plasminogen activator (t-PA) homologs having the fibrinolytic capacity of native t-PA in the presence of fibrin are disclosed. These homologs consist essentially of an amino-terminal fibrin-binding domain and a carboxyl-terminal serine protease domain, wherein said homolog is essentially fibrin-binding inactive in the absence of fibrin, has greater resistance to cleavage by plasmin than native t-PA, and has increased plasma half-life as compared to native t-PA. These homologs are also characterized as having at least one amino acid substitution within four amino acid residues of the activation site corresponding to native t-PA. The DNA constructs, expression vectors, and pharmaceutical compositions relating to these t-PA homologs are also disclosed.

## FIBRINOLYTIC PROTEINS

Technical Field

The present invention relates to fibrinolytic factors in general, and more specifically, to modified tissue-type plasminogen activators.

Background Art

Blood coagulation is a process consisting of a complex interaction of various blood components which eventually gives rise to a fibrin network or clot. Degradation of the fibrin network can be accomplished by activation of the zymogen plasminogen into plasmin, a serine protease which acts directly to degrade the fibrin network. Conversion of plasminogen into plasmin can be catalyzed by tissue-type plasminogen activator (t-PA), which is a fibrin-specific serine protease.

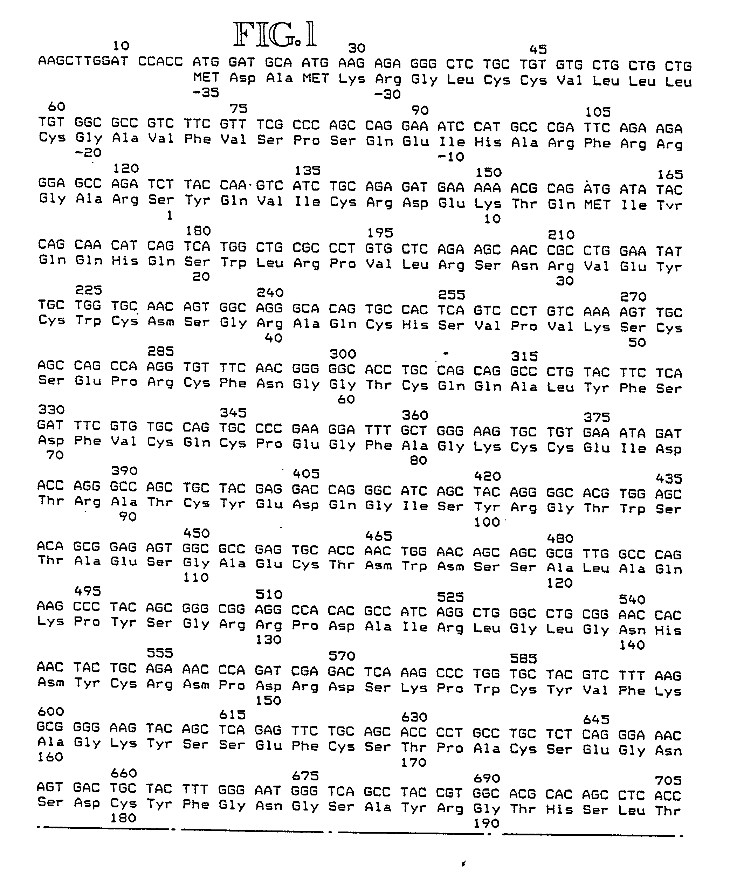

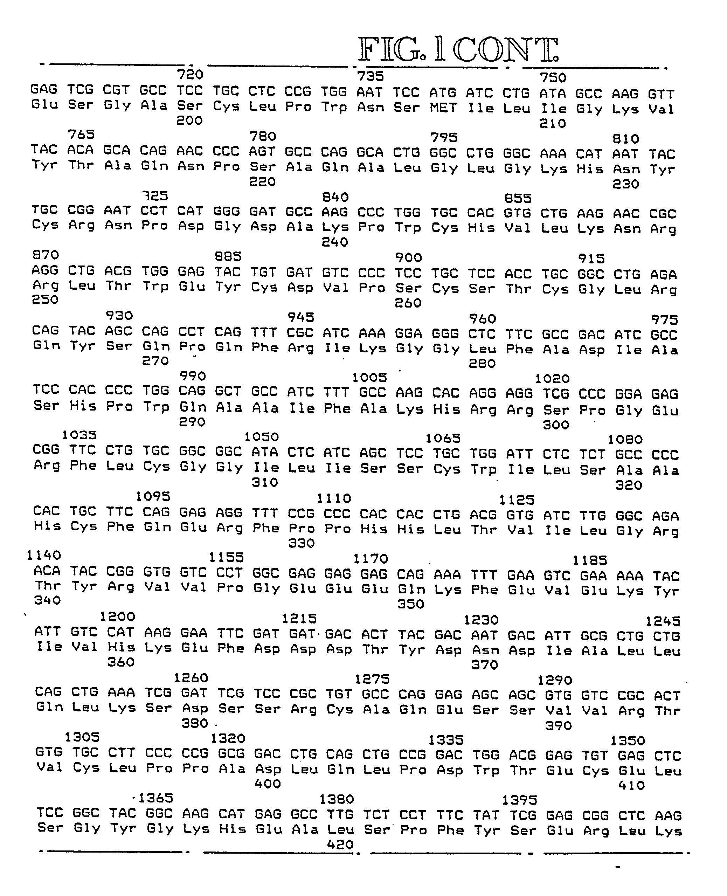

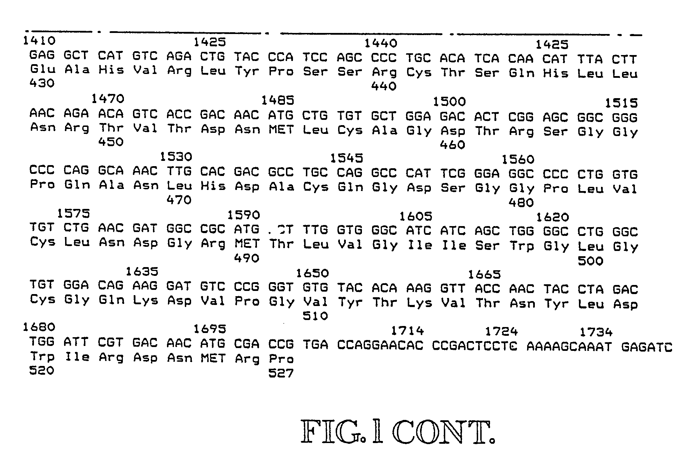

t-PA is believed to be the physiological vascular activator of plasminogen and normally circulates as a single polypeptide chain of $M_r \simeq 72,000$ daltons, which is converted to a two-chain form by cleavage of a peptide bond between amino acids 275 (Arg) and 276 (Ile) with reference to the numbering of amino acids in Figure 1. This cleavage is catalyzed by trypsin or plasmin, and is accompanied by an increase in activity, as measured using synthetic substrates, and by an increase in fibrinolytic activity. The enhancement of fibrinolytic activity of t-PA upon binding to fibrin is due in part to the high concentrations of plasminogen and t-PA present on the surface of a fibrin clot.

The heavy chain of t-PA (two variants of $M_r$ 40,000 and 37,000) is derived from the amino terminus, while the light chain ($M_r$ 33,000) is derived from the carboxy-terminal end of the t-PA molecule.

The amino acid sequence of the potential precursor t-PA protein has been established (Ny et al., PNAS 81: 5355-5359, 1984). From this sequence, a two-dimensional model was generated which contains two triple disulfide structures known as "kringles" in the heavy chain. These kringle structures also occur in prothrombin, plasminogen and urokinase and are believed to be important for binding to fibrin (Ny et al., ibid.). The second kringle ($K_2$) of t-PA is believed to have a higher affinity for fibrin than the first kringle ($K_l$) (Ichinose, Takio, and Fujikawa, J. Clin. Invest. 78:163-169, 1986, and van Zonneveld et al., Proc. Natl. Acad. Sci. USA 83: 4670, 1986).

The heavy chain of t-PA also contains a "finger" domain that is homologous to the finger domains of fibronectin, and a growth factor-like domain, similar to the growth factor domains in epidermal growth factor. The finger domain is involved in the initial binding of t-PA to fibrin. The binding of t-PA to fibrin appears to be a two-stage process (van Zonneveld et al., J. Biol. Chem. 261: 14214, 1986). The initial phase involves relatively weak, specific binding of fibrin to t-PA via the finger domain. Upon initial degradation of fibrin by plasmin, COOH-terminal lysines are formed, leading to increased binding of plasminogen and t-PA via the lysine binding sites in the kringle structures.

t-PA'S light chain contains the active site for serine protease activity, which is highly homologous to the active sites of other serine proteases.

The precursor of native t-PA additionally comprises a pre-region followed downstream by a pro-region, which are collectively referred to as the "pre-pro" region. The pre-region contains a signal peptide which is important for secretion of t-PA by vascular endothelial cells (Ny et al., ibid.). The pre sequence is believed to be responsible for secreting t-PA into the lumen of the endoplasmic reticulum, a necessary step in extracellular secretion, and is removed from the molecule during this process. The pro sequence is believed to be cleaved from the t-PA molecule following transport from the endoplasmic reticulum to the Golgi apparatus.

The use of t-PA for fibrinolysis in animal and human subjects has highlighted several shortcomings of the native molecule. The half-life of t-PA in vivo has been shown to be as short as three minutes in humans (Nilsson et al., Scand. J. Haematol. 33: 49-53, 1984). Injected t-PA was rapidly cleared by the liver, and most of the injected material was metabolized to low molecular weight forms within 30 minutes. This short half-life may limit the effectiveness of t-PA as a thrombolytic agent by necessitating high dosages and prolonged infusion. Cleavage to the two-chain form may also be associated with rapid clearance of t-PA from the bloodstream, but conflicting reports on this have been published (see Wallen et al., Eur. J. Biochem. 132: 681-686, 1983). Fuchs et al. (Blood 65: 539-544, 1985) concluded that infused t-PA is cleared by the liver in a process independent of the proteolytic site, and that infused t-PA will not

2

accumulate in the body. Furthermore, doses of t-PA sufficient to lyse coronary thrombi are far larger than normal physiological levels, and may cause activation of plasminogen throughout the body, leading to systemic degradation of fibrinogen (Sherry, ibid.), which results in dangerous bleeding episodes. This systemic activity may be due to a catalytic activity of the singlechain form of t-PA, activation by a low level of free plasmin in the circulation, or proteolytic activity of the two-chain form of t-PA used in the clinical studies.

Additionally, the production of t-PA using recombinant cells has met with problems which may, in part, result from the presence of active t-PA both intracellularly and in the culture media. t-PA that is produced and secreted by recombinant mammalian cells activates the plasminogen present in serum-containing media to plasmin (a nonspecific protease), which attacks cell matrix proteins and contributes to cell detachment. Intracellular proteins involved in the secretion pathway or in cell adhesion sites may also serve as substrates for t-PA.

Various workers have modified t-PA in attempts to enhance its clinical suitability. Rosa and Rosa (International Patent Application WO 86/01538) modified the Lys at position 277 of t-PA to stabilize the single-chain form of t-PA. Ile (277) t-PA produced in E. coli was found to be less active as a single-chain molecule, as compared to native t-PA. Wallen et al. (ibid.) postulated that this lysine residue may be responsible for proteolytic activity of single-chain t-PA. Genentech (published British Patent Application 2,173,804) discloses amino acid substitutions around the cleavage site of t-PA. A variant t-PA comprising Glu at position 275 was shown to have greater specific activity, as compared to native t-PA. This variant t-PA also formed fewer complexes with t-PA inhibitor. The single-chain form was also shown to have greater affinity for fibrin than the two-chain form. Robinson (WO 84/01786) used enzymatic means to remove carbohydrate side chains from t-PA to increase plasma half-life. Van Zonneveld et al. (Proc. Natl. Acad. Sci. USA 83: 4670-4674, 1986) disclose modified forms of t-PA wherein portions of the heavy chain have been deleted. However, these variant forms of t-PA do not fully overcome the problems associated with the native protein.

There remains a need in the art for a fibrinolytic molecule which has high specificity for activity at the clot site, has long plasma half-life, and facilitates administration. The present invention fulfills this need by providing novel fibrinolytic proteins which contain some of the structural features of native t-PA. Through the use of recombinant DNA technology, a consistent and homogeneous source of these proteins is provided. The proteins can be utilized to lyse existing clots in heart attack and stroke victims, and in others where the need to lyse or suppress the formation of fibrin matrices is therapeutically desirable.

Disclosure of the Invention

Briefly stated, the invention includes a t-PA homolog having the fibrinolytic capacity of native t-PA in the presence of fibrin, consisting essentially of an amino-terminal fibrin-binding domain and a carboxyl-terminal serine protease domain, wherein said homolog is essentially fibrinolytically inactive in the absence of fibrin, has greater resistance to cleavage by plasmin than native t-PA, and has increased half-life as compared to native t-PA.

The preferred embodiments of the t-PA homologs of the present invention include those wherein the amino-terminal fibrinolytically domain comprises at least one or two kringle structures. Where at least two kringle structures are present, one of these may lack carbohydrate. Alternatively, the amino-terminal fibrinolytically domain comprises essentially the K1 and K2 kringle structures of native t-PA, and the K1 kringle structure is modified so as to lack carboydrate.

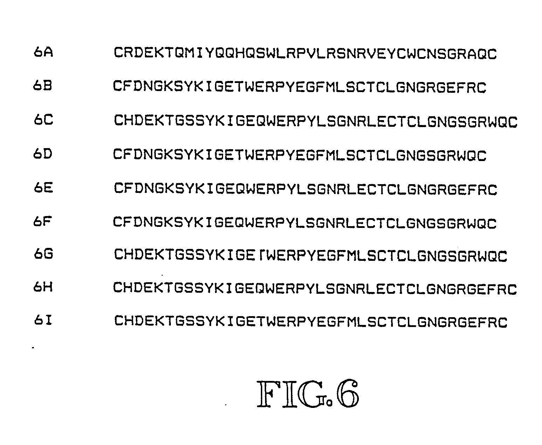

The amino-terminal fibrinolytically domain of the t-PA homologs of the present invention may also comprise at least one finger domain, and these finger domains can have a sequence which is selected from the group consisting of those sequences set forth in Figure 6.

The carboxyl-terminal serine protease domain of the t-PA homologs of the present invention also may be essentially the serine protease domain of native t-PA.

The t-PA homologs may also include amino acid substitutions at various sites. These include Pro, Glu, Gly and Arg at the position corresponding to amino acid 274 of native t-PA; Gly, Leu, Asp and Pro at the position corresponding to amino acid 275 of native t-PA; Leu at the position corresponding to amino acid 277 of native t-PA; and Pro at the position corresponding to amino acid 276 of native t-PA. The Pro (276) homolog exhibits amidolytic activity in the single-chain form and is totally dependent on fibrin for fibrinolytic activity.

Certain of the t-PA homologs of the present invention are characterized as being activated by cleavage by thrombin.

A further embodiment of the present invention includes DNA constructs encoding the aforementioned t-PA homologs which have the fibrinolytic capacity of native t-PA in the presence of fibrin, wherein said DNA constructs have a nucleotide sequence consisting essentially of a first region, encoding a fibrin-binding domain, and a second region positioned downstream of said first region, said second region encoding a catalytic domain for serine protease activity of native t-PA, wherein said nucleotide sequence encodes a t-PA homolog having at least one amino acid substitution at an amino acid selected from the group consisting of amino acids corresponding to amino acids number 274, 275, 276 and 277 of native t-PA.

A still further embodiment of the present invention includes expression vectors capable of directing the expression of the aforementioned t-PA homologs. These homologs are characterized as having the fibrinolytic capacity of native t-PA in the presence of fibrin, wherein said vector includes a promoter, said promoter being operably linked to a nucleotide sequence, said nucleotide sequence encoding said homolog and consisting essentially of a first region, encoding a fibrin-binding domain, and a second region positioned downstream of said first region, said second region encoding a catalytic domain for the serine protease activity of native t-PA. These t-PA homologs are characterized as having at least one amino acid substitution at an amino acid selected from the group consisting of amino acids corresponding to amino acids number 274, 275, 276 and 277 of native t-PA.

Additionally, the present invention includes a pharmaceutical composition comprising a t-PA homolog having the fibrinolytic capacity of native t-PA in the presence of fibrin, consisting essentially of an amino-terminal fibrin-binding domain and a carboxyl-terminal serine protease domain, said homolog having at least one amino acid substitution within four amino acid residues of the activation site and wherein said homolog is essentially fibrinolytically inactive in the absence of fibrin, has a greater resistance to cleavage by plasmin than native t-PA, and has increased plasma half-life as compared to native t-PA. This homolog is delivered in combination with a pharmaceutically acceptable carrier or diluent.

Other aspects of the invention will become evident upon reference to the following detailed description and attached drawings.

## Brief Description of the Drawings

Figure 1 illustrates the pre-pro t-PA coding sequence constructed from cDNA and synthesized oligonucleotides, together with the amino acid sequence of the encoded protein. Numbers above the lines refer to nucleotide position and numbers below the lines refer to amino acid position.

Figure 2 illustrates the construction of the vector Zem99.

Figure 3 illustrates the construction of the t-PA expression vector pDR817.

Figure 4 shows a comparison of the amino-termini of several modified t-PA molecules described herein.

Figure 5 illustrates the construction of plasmid pDR3002 and t-PA vectors derived from pDR3002.

Figure 6 illustrates the amino acid sequences of "consensus" finger domains.

Figure 7 illustrates the amino acid sequence of a mutant t-PA protein lacking the finger and growth factor domains, and the nucleotide sequence encoding the protein.

Figure 8 illustrates the amino acid sequence of a mutant t-PA protein lacking the finger, growth factor, and Kringle 1 domains, together with the nucleotide sequence encoding the protein.

Figure 9 illustrates the construction of the plasmid Zem182b.

Figure 10 illustrates the construction of the pMH series of expression vectors, comprising DNA sequences encoding t-PA homologs with altered activation sites.

Figure 11 illustrates the construction of the expression vectors p820a and p820b.

Figure 12 illustrates the construction of the vector Zem219b.

Figure 13 illustrates the results of a fibrin stimulation assay on native t-PA and t-PA homologs.

Figure 14a illustrates the results of amidolytic activity assays on one-chain and two-chain forms of native t-PA and a t-PA homolog in the absence of fibrin.

Figure 14b illustrates the results of plasminogen activation assays on one-chain and two-chain forms of native t-PA and a t-PA homolog in the presence of fibrin.

## Best Mode for Carrying Out the Invention

Prior to setting forth the invention, it may be helpful to an understanding thereof to set forth definitions of certain terms used herein.

## Complementary DNA or cDNA:

A DNA molecule or sequence which has been enzymatically synthesized from the sequences present in an mRNA template.

## DNA construct:

A DNA molecule, or a clone of such a molecule, either single- or double-stranded; which may be isolated in partial form from a naturally occurring gene or which has been modified to contain segments of DNA which are combined and juxtaposed in a manner which would not otherwise exist in nature.

## Plasmid or vector:

A DNA construct containing genetic information which provides for its replication when inserted into a host cell. Replication may be autonomous or achieved by integration into the host genome. A plasmid generally contains at least one gene sequence to be expressed in the host cell, as well as sequences encoding functions which facilitate such gene expression, including promoters and transcription initiation sites and terminators. It may be a linear molecule or a closed, circular molecule.

## Pre-pro region:

An amino acid sequence which generally occurs at the amino termini of the precursors of certain proteins, and which is generally cleaved from the protein, at least in part, during secretion. The pre-pro region comprises, in part, sequences directing the protein into the secretory pathway of the cell.

## Domain:

A three-dimensional, self-assembling array of amino acids of a protein molecule, which contains structural elements necessary for a specific biological activity of that protein.

## Fibrin-binding domain:

That portion of a protein necessary for the binding of that protein to fibrin. For example, in native t-PA, the finger domain and kringle structures individually and collectively contribute to the fibrin binding. According to the present invention, it has been found that the EGF domain does not significantly contribute to fibrin binding of t-PA but does contribute to the very short plasma half-life of t-PA.

## Fibrinolytic capacity:

The ability of a molecule to stimulate the degradation of fibrin in vivo or in vitro. As used herein, "fibrinolytic capacity of t-PA" shall mean the conversion, in the presence of fibrin, of the pro-enzyme or zymogen plasminogen into plasmin, which in turn degrades fibrin matrices. Because fibrin acts as a cofactor in the activation of plasminogen, native t-PA has reduced fibrinolytic capacity in the absence of fibrin.

## t-PA homolog:

A protein having functional homology to native t-PA. Such a protein will have at least a fibrin-binding portion and a serine protease portion. These functions are believed to be associated with the primary and secondary structures of the respective portions of the protein homologs (as well as native t-PA). t-PA homologs may be fibrin-binding active as single-chain molecules or may be activated upon proteolytic cleavage at an activation site between the fibrin-binding and serine protease portions. The amino acid sequence around the activation site will be that of native t-PA, with at least one amino acid substitution within fifteen amino acid residues of the activation site and preferably within four amino acid residues of the activation site. As used herein, the term "activation site" shall mean that point in a t-PA homolog corresponding to the peptide bond between amino acids 275 and 276 in native t-PA. In some homologs, cleavage of this bond is not a prerequisite for fibrinolytic capacity.

As noted above, t-PA is known to play a major role in the degradation of fibrin clots. However, in clinical applications it would be advantageous to employ fibrinolytic agents possessing enhanced fibrin-binding capability, increased biological half-life, increased solubility, and/or increased specificity for fibrin as compared to native t-PA. Such agents would preferably lack structural and functional features which may not play an active role in fibrinolysis.

In light of the facts that native t-PA is a composite mosaic polypeptide and that the t-PA gene comprises multiple exons encoding the aforementioned structural and functional domains (Ny et al., ibid.), it is possible to analyze the individual and collective contributions of these domains to the overall function of the molecule. This information can then be applied to the design of fibrinolytic proteins having a high clot specificity, long plasma half-life, and other desirable characteristics. It is preferred to produce such proteins through the application of recombinant DNA technology. Optimization of the protein product can then be accomplished by cloning only nucleotide sequences which encode the desired structural and functional properties, while eliminating those sequences which do not contribute to the desired biological activity or contribute to undesirable side effects. Mul tiple copies of the desired structures may also be incorporated into the optimized proteins. Based on an analysis of t-PA and related molecules, amino acid sequences may be modified or substituted with related sequences from other proteins to further enhance their desired properties.

For example, it would be desirable to produce a fibrinolytic molecule which is essentially inactive in the absence of fibrin but which is as active as native t-PA in the presence of fibrin and which furthermore does not contain all or part of the growth factor (GF) domain of native t-PA, since it has been discovered that an intact, properly folded GF domain is not necessary for fibrinolytic activity and may be involved in one of the mechanisms regulating t-PA plasma levels. Plasma half-life may also be enhanced by altering the structure of the growth factor domain through site-specific mutagenesis. Deletion of all or part of the growth factor domain may also increase the solubility of the molecule, due to the hydrophobic nature of that domain. Increased solubility would permit use of smaller (injectable) volumes of fibrinolytic agents and permit faster administration of these agents to patients. Elimination of several extraneous domains or structural features has been found to increase the half-life of the resultant molecule in vivo without substantially altering fibrinolytic capacity. Specific activity may also be increased, thereby allowing the use of smaller doses. Furthermore, enhancement of fibrin binding may be achieved by modifying naturally-occurring fibrin-binding domains to increase their affinity for a fibrin clot or by adding additional kringle structures and/or finger domains.

As noted above, the fibrinolytic proteins of the present invention consist essentially of an amino terminal fibrin-binding domain and a carboxyl terminal serine protease domain. As such, these proteins embody the essential functional features of native t-PA. The structural elements responsible for these functions are modified, however, so as to enhance the desirable activities found in native t-PA while minimizing its undesirable features. One particularly desirable feature of these proteins is their high clot specificity as compared to that of native t-PA. This specificity is achieved through modifications of the activation site, resulting in a protein which has fibrinolytic capacity almost exclusively in the presence of a fibrin clot. Thus, the proteins of the present invention provide such advantages as increased specificity (i.e., decreased systemic activity), increased plasma half-life, and increased solubility. Specific activity may also be increased.

The amino acid sequences around the activation sites of these proteins are derived from the sequence of the activation site of native t-PA. Through the use of amino acid deletions, amino acid substitutions, or combinations of amino acid deletions and substitutions, a molecule is constructed which is resistant to cleavage in the absence of fibrin but has the fibrinolytic capacity of t-PA in the presence of fibrin. In one embodiment, the protein is activated upon cleavage by thrombin. In another embodiment, the molecule has clot-specific fibrinolytic activity in both the single-chain and two-chain forms. In a third embodiment, the t-PA homolog has activity in the single-chain form but remains uncleaved in the presence of fibrin.

The amino acid changes of the present invention are of four groups, those at amino acid position 274, those at position 275, those at position 276 and those at position 277. Amino acid 274 (Phe) is preferably replaced by Pro, Glu, Gly or Arg, most preferably Pro. Amino acid 275 (Arg) is preferably replaced by Gly, Leu, Asp or Pro; Ile (276) is preferably replaced by Pro; and Lys (277) is preferably replaced by Leu. The changes at amino acid 275, with or without additional changes at position 277, result in molecules which are much more resistant to cleavage by plasmin than is native t-PA. These proteins may require high concentrations of plasmin for conversion to the two-chain form. Furthermore, the change to proline at amino acid 274 will allow cleavage of the molecule by thrombin. This is expected to increase the clot specificity over that of native t-PA due to the high levels of thrombin expected to be present at clots. Increased specificity should also facilitate the production of t-PA by transfected mammalian cells. These substitutions may be made individually or in combination. Proteins having amino acid substitutions in the region of amino acids 274-277 may be fibrin-binding active in the single chain form.

The fibrin-binding domains of these proteins are designed to enhance fibrin binding over that observed for native t-PA. Characteristic of these proteins is the deletion of regions of the t-PA heavy chain which do not contribute to fibrin binding. For example, it has been found that the growth factor domain does not substantially contribute to fibrin binding, and that all of that domain may be eliminated without destroying fibrinolytic capacity. The Kringle 1 domain can also be eliminated. The remaining sequences which constitute the fibrin-binding domain may be those found in native t-PA or may be modified by substituting specific amino acids within the fibrin-binding domain and/or by duplicating structural elements known to contribute to fibrin binding, such as the finger domain or Kringle 2 region.

A portion of the fibrin-binding domain which is preferred for additional modification is the finger domain. As previously noted, the initial binding of t-PA to fibrin appears to be dependent on the finger domain. By increasing the fibrin affinity of the finger domain, the binding strength and specificity of a fibrinolytic protein may be enhanced. According to the present invention, three strategies are used to enhance the affinity of the finger domain for fibrin: replacement of the t-PA finger domain with a fibrin-binding region of fibronectin; construction of a fibrinolytic protein comprising a consensus fibrin-binding finger domain (designated Fcon), the sequence of which is based on an analysis of the finger domains of fibronectin; and duplication of either the Fcon or native t-PA finger regions. The consensus finger domain will preferably be constructed so as to eliminate the potential proteolytic cleavage sites present in t-PA at Arg-27, Lys-49, and Arg-89. Particularly preferred molecules in this regard have the structure Fcon-Kringle 2-serine protease or combine the Fcon with the Phe (274) to Pro mutation.

The fibrinolytic proteins of the present invention may be fully glycosylated, partially glycosylated, or unglycosylated. Native t-PA is a glycoprotein containing carbohydrate chains linked to asparagine residues 117, 184 and 448. Although carbohydrate can be removed from a protein by enzymatic means, such as that disclosed by Robinson (WO 8401786), it is preferred that this be accomplished by modifying the amino acid sequence at the glycosylation site through the use of site-specific mutagenesis of a cloned DNA sequence (Zoller et al., DNA 3: 479-488, 1984). This permits the production of a protein in which some or none of the naturally-occurring glycosylation sites are functional. The N-linked glycosylation sites of t-PA are generally characterized by the amino acid sequence Asn-X-Ser/Thr, where X is any amino acid except Pro. To prevent glycosylation, this sequence is preferably altered to Gln-X-Ser/Thr or Asn-X-Y, where Y is any amino acid except Ser or Thr. Preferred proteins described herein lack carbohydrate on the Kringle 1 domain. A particularly preferred protein has the structure Fcon-Kringle 1-Kringle 2-serine protease, wherein the Kringle 1 lacks carbohydrate and the activation site is modified as described above.

According to the present invention, it is preferred to produce these novel proteins through the use of recombinant DNA technology, using cDNA clones or genomic clones as starting materials. Suitable DNA sequences can also be synthesized according to standard procedures. It is preferred to use cDNA clones because, by employing the full-length cDNA encoding native t-PA as starting material for producing modified t-PA, introns are removed so that all exons of the native t-PA are present and correctly oriented with respect to one another. Full-length cDNA also provides the advantage of easily generating modifications in the fibrin-binding domain by deleting portions of the t-PA cDNA from the 5' end, thus providing a multiplicity of cDNA fragments which can be ultimately inserted into host cells. The cDNA can also be used as a template for deletion, alteration or insertion of sequences via oligonucleotide-directed mutagenesis.

Recombinant DNA technology allows the convenient enhancement of the fibrin-binding domain of native t-PA by the insertion of additional kringle structures, additional finger domains, or substitution of the finger domain. This methodology provides a means for selecting the optimum combination of functional domains found in native t-PA or in related proteins, and thus provides fibrinolytic agents with enhanced biological activity with respect to fibrin-binding and specificity of serine protease activity.

Amino acid changes are introduced by site-specific mutagenesis using a cloned t-PA cDNA fragment as a template. The mutated sequence is then joined to the remainder of the t-PA coding sequence, and the reconstructed coding sequence is then inserted into an expression vector. The mutant sequences may be expressed in various host cells, including mammalian cells, yeast and other fungi, and bacteria. Cultured mammalian cells are preferred. A particularly preferred mammalian cell line is the BHK cell line tk⁻ts13 (Waechter and Baserga, Proc. Natl. Acad. Sci. USA 79: 1106-1110, 1982). Methods for expressing cloned genes in each of these hosts are known in the art.

Accordingly, the present invention provides a method of producing novel proteins having biological activity that is substantially the same as t-PA. Novel proteins described herein have been shown to have up to ten-fold greater in vivo half-life than native t-PA.

A cDNA clone containing sequences encoding mature human t-PA has been previously identified. The t-PA cDNA sequence has been inserted into plasmid pDR1296, and this plasmid has been introduced into E. coli JM83. This transformant has been deposited with the American Type Culture Collection, Bethesda, Md., and assigned Accession No. 53347.

Strain pDR1296/JM83 was used as the source of t-PA cDNA. Plasmid pDR1296 was isolated, and the t-PA cDNA was excised by restriction endonuclease digestion. Three approaches may be followed for removing structural domains which are not needed for fibrinolytic activity. In the first approach, the t-PA restriction fragment was incubated with Bal 31, and the enzymatic reaction time was controlled in order to produce a continuum of t-PA nucleotide sequences which exhibit size heterogeneity. This size heterogeneity results from the progressive shortening of the 5' terminus of the t-PA cDNA. The Bal 31 digested fragments were separated by gel electrophoresis, and the desired fragment size range was selected. The fragments were eluted from the gel according to conventional techniques. In a second approach, part of the cDNA sequence from pDR1296 was used as a template for oligonucleotide-directed deletion mutagenesis. Techniques for synthesizing oligonucleotides are well known in the art. By this method, sequences encoding specific domains of native t-PA are precisely deleted or altered. In a third approach, the 5' coding region of a modified t-PA sequence may be constructed from synthesized oligonucleotides and joined to the 3' region of the cDNA.

A DNA fragment comprising the activation site was then cloned into an M13 vector, and the amino acid sequence around the activation site was modified by site-specific mutagenesis.

Complete DNA sequences encoding the fibrinolytic proteins were then constructed by combining a 5' coding fragment encoding the fibrin-binding domain, a fragment encoding the modified activation site, and a fragment encoding the COOH-terminal portion of the protein. In a preferred embodiment, an appropriate pre-pro sequence was also included to allow secretion of the protein into the medium in order to facilitate purification of the recombinant protein. The pre-pro sequence of t-PA may be isolated from cDNA or genomic libraries, or may be constructed from synthesized oligonucleotides. Oligonucleotides are preferably machine-synthesized, purified, and annealed to construct double-stranded fragments. The resultant double-stranded fragments are ligated as necessary to produce the pre-pro sequence. This pre-pro sequence comprises a 105 bp sequence, as depicted in Figure 1. The synthesized pre-pro sequence was then joined to the 5' termini of the modified t-PA fragments. Other pre-pro sequences may be used, depending on the host cell type selected. In some instances it may be desirable to use a pre-pro sequence which is endogenous to the host species.

The pre-pro-t-PA fragments are inserted into a suitable expression vector, which in turn is inserted into appropriate host cells. The method of insertion will depend upon the particular host cell chosen. Methods for transfecting mammalian cells and transforming bacteria and fungi with foreign DNA are well known in the art. Suitable expression vectors will comprise a promoter which is capable of directing the transcription of a foreign gene in a host cell and a functional transcription termination site.

In some instances, it is preferred that expression vectors further comprise an origin of replication, as well as sequences which regulate and/or enhance expression levels, depending on the host cell selected. Suitable expression vectors may be derived from plasmids, RNA and DNA viruses or cellular DNA sequences, or may contain elements of each.

Preferred prokaryotic hosts for use in carrying out the present invention are strains of the bacteria Escherichia coli, although Bacillus and other genera are also useful. Techniques for transforming these hosts, and for expressing foreign DNA sequences cloned in them, are well known in the art (see, for example, Maniatis et al., Molecular Cloning: A Laboratory Manual, Cold Spring Harbor Laboratory, 1982). Vectors used for expressing foreign DNA in bacterial hosts will generally contain a selectable marker, such as a gene for antibiotic resistance, and a promoter which functions in the host cell. Appropriate promoters include the trp (Nichols and Yanofsky, Meth. in Enzymology 101: 155, 1983), lac (Casadaban et al., J. Bact. 143: 971-980, 1980), TAC (Russell et al., Gene 20: 231-243, 1982), and phage λ promoter systems. Plasmids useful for transforming bacteria include pBR322 (Bolivar et al., Gene 2: 95-113, 1977), the pUC plasmids (Messing, Meth. in Enzymology 101: 20-77, 1983; and Vieira and Messing, Gene 19: 259-268, 1982), pCQV2 (Queen, J. Mol. Appl. Genet. 2: 1-10, 1983), and derivatives thereof.

Eukaryotic microorganisms, such as the yeast Saccharomyces cerevisiae, or filamentous fungi including Aspergillus, may also be used as host cells. Particularly preferred species of Aspergillus include A.

8

nidulans, A. niger, A. oryzae, and A. terreus. Techniques for transforming yeast are described by Beggs (Nature 275: 104-108, 1978). Expression vectors for use in yeast include YRp7 (Struhl et al., Proc. Natl. Acad. Sci. USA 76: 1035-1039, 1979), YEp13 (Broach et al., Gene 8: 121-133, 1979), pJDB248 and pJDB219 (Beggs, ibid.), and derivatives thereof. Such vectors will generally comprise a selectable marker, such as the nutritional marker TRP, which allows selection in a host strain carrying a trp1 mutation. Preferred promoters for use in yeast expression vectors include promoters from yeast glycolytic genes (Hitzeman et al., J. Biol. Chem. 255: 12073-12080, 1980; Alber and Kawasaki, J. Mol. Appl. Genet. 1: 419-434, 1982) or alcohol dehydrogenase genes (Young et al., in Genetic Engineering of Microorganisms for Chemicals, Hollaender et al., eds., p. 335, Plenum, New York, 1982; and Ammerer, Meth. in Enzymology 101: 192-201, 1983). To facilitate purification of a modified t-PA protein produced in a yeast transformant and to obtain proper disulphide bond formation, a signal sequence from a yeast gene encoding a secreted protein may be substituted for the t-PA pre-pro sequence. A particularly preferred signal sequence is the pre-pro region of the MFα1 gene (Kurjan and Herskowitz, Cell 30: 933-943, 1982; and Singh (EP 123,544)). Aspergillus species may be transformed according to known procedures, for example, that of Yelton et al. (Proc. Natl. Acad. Sci. USA 81: 1740-1747, 1984).

Higher eukaryotic cells may also serve as host cells in carrying out the present invention. Cultured mammalian cells, such as the BHK, CHO, NS-1, SP2/0, and J558L cell lines, are preferred. Tk⁻BHK cells are a particularly preferred adherent cell line. Expression vectors for use in mammalian cells will comprise a promoter capable of directing the transcription of a cloned gene introduced into a mammalian cell. Particularly preferred promoters include the SV40 promoter (Subramani et al., Mol. Cell Biol. 1: 854-64, 1981), the MT-1 promoter (Palmiter et al., Science 222: 809-814, 1983), and the mouse kappa gene promoter (Bergman et al., Proc. Natl. Acad. Sci. USA 81: 7041-7045, 1984). Also contained in the expression vectors is a transcription terminator, located downstream of the insertion site for the DNA sequence to be expressed. A preferred terminator is the human growth hormone (hGH) gene terminator (DeNoto et al., Nuc. Acids Res. 9: 3719-3730, 1981). In addition, vectors will preferably contain enhancer sequences appropriate to the particular host cell line.

For expression of mutant t-PAs in cultured mammalian cells, expression vectors containing cloned t-PA sequences are introduced into the cells by appropriate transfection techniques, such as calcium phosphate-mediated transfection (Graham and Van der Eb, Virology 52: 456-467, 1973; as modified by Wigler et al., Proc. Natl. Acad. Sci. USA 77: 3567-3570, 1980) or electroporation (Neumann et al. EMBO J. 1: 841-845, 1982). A DNA-calcium phosphate precipitate is formed, and then applied to the cells in the presence of medium containing chloroquine (100 μm). The cells are incubated for four hours with the precipitate, followed by a two-minute, 15% glycerol shock. A portion of the cells take up the DNA and maintain it inside the cell for several days. A small fraction of the cells integrate the DNA into the genome of the host cell or maintain the DNA in non-chromosomal nuclear structures. These transfectants can be identified by cotransfection with a gene that confers a selectable phenotype (a selectable marker). Preferred selectable markers include the DHFR gene, which imparts cellular resistance to methotrexate (MTX), an inhibitor of nucleotide synthesis; or the bacterial neomycin resistance gene, which confers resistance to the drug G-418, an inhibitor of protein synthesis. After the host cells have taken up the DNA, drug selection is applied to select for a population of cells that are expressing the selectable marker at levels high enough to confer resistance.

Coamplification as a means to increase expression levels can be accomplished by the addition of high concentrations of MTX to the culture medium at the time of the initial selection, or subsequently accomplished by sequentially increasing the concentration of MTX in the medium, followed by repeated cloning by dilution of the drugresistant cell lines. Variations exist in the ability to amplify and relate both to the initial genomic configuration (i.e., extra-chromosomal vs. chromosomal) of the cotransfected DNA sequences and to the mechanism of amplification itself, in which variable amounts of DNA rearrangements can occur. This is noticed upon further amplification of clones which have been previously shown to be stable. For this reason, it is necessary to clone by dilution after every amplification step. Cells which express the DHFR marker are then selected and screened for production of t-PA. Screening may be done by enzyme linked immunosorbent assay (ELISA) or by biological activity assays.

Additionally, it has been observed that under certain conditions, production of t-PA has a deleterious effect on mammalian cells in culture. This is believed to be due in part to the plasmin (a nonspecific protease) generated when cells which have been transfected to produce t-PA are cultured in media containing plasminogen, a component of serum. The t-PA produced by the transfected cells activates the plasminogen to plasmin, which then attacks cell membranes and contributes to cell detachment. Other proteolytic activities are also believed to be involved. It has been found that removing serum plasminogen by passage over a lysine sepharose column, and including protease inhibitors in the culture media, blocks the activation of plasminogen and facilitates t-PA production. A particularly preferred protease inhibitor is aprotinin, which is included in the culture media at a concentration of from approximately 100 units/ml to 50,000 units/ml, preferably 100 units/ml, in medium containing plasminogen-free serum, or from approximately 1000 units/ml to 50,000 units/ml, preferably 1000 units/ml, if using normal fetal calf serum. Additional useful protease inhibitors include transexamic acid and epsilon amino caproic acid, which are included in the media at mM concentrations.

The t-PA thus produced is recovered from the cultured cells by removing the culture medium and fractionating it. A preferred method of fractionation is affinity chromatography using an anti-t-PA antibody. Other conventional purification methods, such as ion-exchange chromatography, high-performance liquid chromatography or gel filtration, may also be used.

The proteins of the present invention may be used within pharmaceutical compositions for the treatment of thrombosis. The pharmaceutical compositions will comprise these proteins in combination with a carrier or diluent, such as sterile water or sterile saline, and may also com prise appropriate excipients and/or solvents. The resulting aqueous solutions may be packaged for use or filtered under aseptic conditions and lyophilized, the lyophilized preparation being combined with a sterile aqueous solution prior to administration. Administration is preferably by injection.

In summary, the present invention provides modified t-PA proteins and a method for production of these modified t-PA proteins which have substantially the same biological activity as native t-PA, through use of stably transfected or transformed host cells. The protein products thus expressed are then purified from the cells or cell growth media and assayed for biological activity. The assay may monitor conversion of plasminogen to plasmin, clot lysis ability, fibrin-binding affinity or plasma half-life characteristics. Immunological assays may also be used.

To summarize the examples which follow, Example 1 discloses a t-PA cDNA clone. The cDNA is combined with a synthesized pre-pro sequence, and the resultant full-length coding sequence is used to construct the vector Zem99.

Example 2 describes the preparation of t-PA sequences having random 5' end deletions.

Example 3 describes the construction of DNA sequences encoding t-PA molecules with modified finger domains.

Example 4 describes the deletion of coding sequences for the finger domain and growth factor domain of t-PA.

Example 5 describes the deletion of the coding sequences for the finger domain, growth factor domain, and Kringle 1 structure of t-PA.

Example 6 describes the elimination of carbohydrate addition sites on the fibrinolytic proteins by site-specific mutagenesis of cDNA.

Example 7 describes the deletion of the growth factor domain coding sequence from the t-PA cDNA.

Example 8 discloses the site-specific mutagenesis of the t-PA activation site.

Example 9 discloses novel proteins having specific fibrinolytic activity.

Example 10 discloses methods for the production of fibrinolytic proteins using transfected mammalian cells.

Example 11 discloses the purification and characterization of the fibrinolytic proteins produced as described in Example 10.

<div align="center">EXAMPLES</div>

Example 1 - Construction of a Full-Length t-PA Clone

The sequence of a native human t-PA cDNA clone has been reported (Pennica et al., Nature 301: 214-221, 1983). The sequence encodes a pre-pro peptide of 32-35 amino acids followed by a 527-530 amino acid mature protein.

A cDNA clone comprising the coding sequence for mature t-PA was constructed using as starting material mRNA from the Bowes melanoma cell line (Rijken and Collen, J. Biol. Chem. 256: 7035-7041, 1981). This cDNA was then used to construct the plasmid pDR1296. Escherichia coli strain JM83 transformed with pDR1296 has been deposited with the American Type Culture Collection under Accession No. 53347.

<div align="center">10</div>

Because the pre-pro sequence was not present in the cDNA clone pDR1296, it was constructed from synthesized oligonucleotides and subsequently joined to the cDNA. In the synthesized t-PA pre-pro sequence, cleavage sites for Bam HI and Nco I were introduced immediately 5′ to the first codon (ATG) of the pre-pro sequence, and a Bgl II (Sau 3A, Xho II) site was maintained at the 3′ end of the pre-pro sequence. The naturally-occurring pre-pro sequence lacks a convenient restriction site near the middle; however, the sequence GGAGCA (coding for amino acids -20 and -19, Gly-Ala) can be altered to GGCGCC to provide a Nar I site without changing the amino acid sequence.

To construct the pre-pro sequence, the following oligonucleotides were synthesized using an Applied Biosystems Model 380-A DNA synthesizer:

ZC131:  $^5$′GGA TCC ATG GAT GCA ATG AAG AGA GGG CTC TGC TGT GTG$^3$′

ZC132:  $^5$′TGG CGC CAC ACA GCA GCA GCA CAC AGC AGAG$^3$ ,

ZC133:  $^5$′GGC GCC GTC TTC GTT TCG CCC AGC CAG GAA ATC CATG$^3$ ,

ZC134:  $^5$ AGA TCT GGC TCC TCT TCT GAA TCG GGC ATG GAT TTC CT$^3$

Following purification, oligomers ZC131 and ZC132 were annealed to produce an overlap of 12 base pairs (Section 1). Oligomers ZC133 and ZC134 were similarly annealed (Section 2).

The oligomers were mixed in Pol I buffer (Bethesda Research Labs), heated to 65° C for five minutes, and slowly cooled to room temperature for four hours to anneal. Ten units of DNA polymerase I were added and the reaction proceeded for two hours at room temperature. The mixtures were electrophoresed on an 8% polyacrylamide-urea sequencing gel at 1,000 volts for 2½ hours in order to size fractionate the reaction products. The correct size fragments (those in which the polymerase reaction went to completion) were cut from the gel and extracted.

After annealing, Section 1 was cut with Bam HI and Nar I and cloned into Bam HI + Nar I - cut pUC8 (Vieira and Messing, Gene 19: 259-268, 1982; and Messing, Meth. in Enzymology 101: 20-77, 1983). Section 2 was reannealed and cut with Nar I and Bgl II and cloned into Bam HI + Nar I (Sau 3A, Xho I) - cut pUC8. Colonies were screened with the appropriate labeled oligonucleotides. Plasmids identified as positive by colony hybridization were sequenced to verify that the correct sequence had been cloned.

Section 1 was then purified from a Bam HI + Nar I double digest of the appropriate pUC clone. Section 2 was purified from a Nar I + Xho II digest. The two fragments were joined at the Nar I site and cloned into Bam HI cut pUC8.

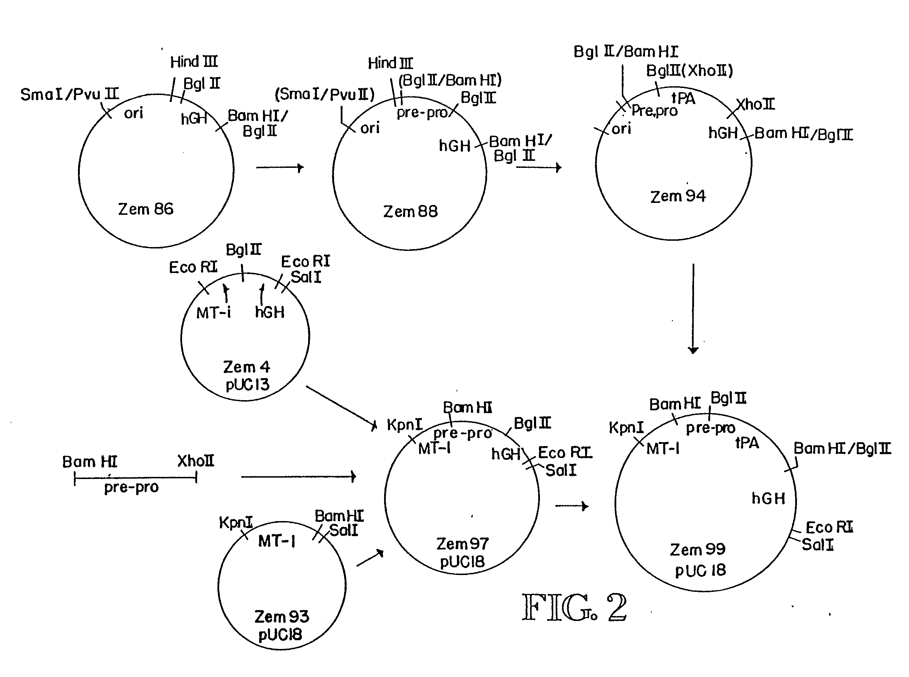

The t-PA sequence of pDR1296 was then joined to the synthesized pre-pro sequence in the following manner (Figure 2). Plasmid pIC19R (Marsh et al., Gene 32: 481-486, 1984) was digested with Sma I and Hind III. The ori region of SV40 from map position 270 (Pvu II) to position 5171 (Hind III) was then ligated to the linearized pIC19R to produce plasmid Zem67. This plasmid was then cleaved with Bgl II and the terminator region from the human growth hormone gene (De Noto et al., Nuc. Acids Res. 9: 3719-3730, 1981) was inserted as a Bgl II-Bam HI fragment to produce plasmid Zem86. The synthesized t-PA pre-pro sequence was removed from the pUC8 vector by digestion with Bam HI and Xho II. This fragment was inserted into Bgl II-digested Zem86 to produce plasmid Zem88. Plasmid pDR1296 was digested with Bgl II and Bam HI, and the t-PA cDNA fragment was isolated and inserted into Bgl II - cut Zem88. The resultant plasmid was designated Zem94.

The vector Zem99, comprising the MT-1 promoter, complete t-PA coding sequence, and the hGH terminator, was then assembled in the following manner (Figure 2). A Kpn I-Bam HI fragment comprising the MT-1 promoter was isolated from MThGH111 (Palmiter et al., Science 222: 809-814, 1983) and inserted into pUC18 to construct Zem93. Plasmid MThGH112 (Palmiter et al., ibid.) was digested with Bgl II and re-ligated to eliminate the hGH coding sequence. The MT-1 promoter and hGH terminator were then isolated as an Eco RI fragment and inserted into pUC13 to construct Zem4. Zem93 was then linearized by digestion with Bam HI and Sal I. Zem4 was digested with Bgl II and Sal I and the hGH terminator was purified. The t-PA pre-pro sequence was removed from the pUC8 vector as a Bam HI-XhO II fragment. The three DNA fragments were then joined and a plasmid having the structure of Zem97 (Figure 2) was selected. Zem97 was cut with Bgl II and the Xho II t-PA fragment from Zem94 was inserted. The resultant vector is Zem99.

Example 2 - Preparation of t-PA Sequences Having Random 5′ End Deletions

## A. Deletion of 5' Coding Sequences

Ten μg of pDR1296 were digested with either five units of Bgl II or five units of Nar I to completion. The resultant linearized DNAs were electrophoresed on a 0.7% agarose gel. The DNA fragments were eluted from the gel using TE buffer (10 mM Tris, pH 7.4, 5 mM EDTA). The DNA fragments were resuspended in 46 μl of $H_2O$ and 12 μl of 5x Ba131 buffer (3M NaCl, 62.5 mM $CaCl_2$, 62.5 mM $MgCl_2$, 100 mM Tris-Hcl, pH 8.0, 50 mM EDTA, pH 8.0) plus 2 μl of Ba131 (0.5 units/μl, obtained from Bethesda Research Laboratories). The reaction mixtures were incubated at 30°C and 10 μl aliquots were removed at one minute intervals for six minutes. The digested samples were combined, extracted with phenol-$CHCl_3$, and precipitated with ethanol. The fragment ends were filled in with DNA polymerase I (Klenow fragment) and dNTPS. Following the fill-in reaction, samples were digested with Xba I and separated on a 0.7% agarose gel. Fragments smaller than 1700 bp were cut out and extracted with TE buffer. Subsequent mapping identified 55 truncated sequences.

## B. Preparation of pDR817 Expression Vector

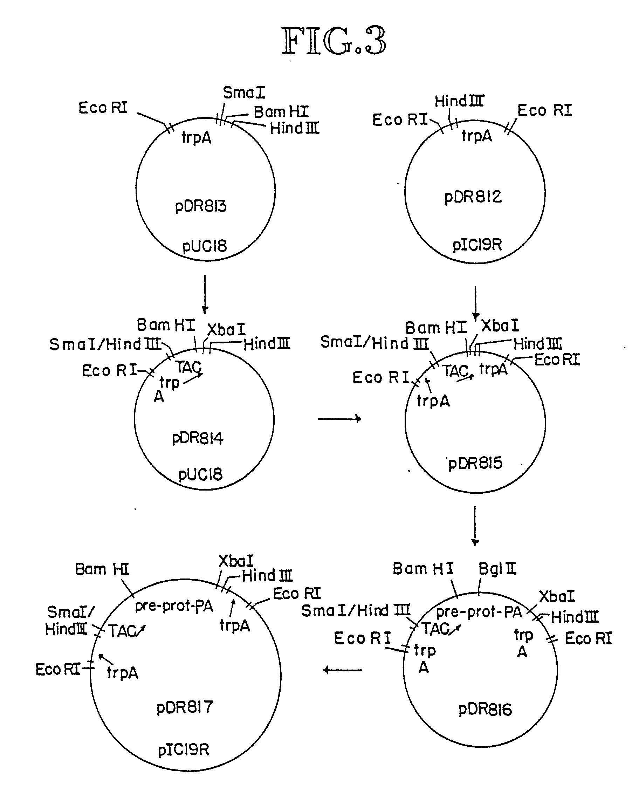

Referring to Figure 3, the 28 bp trpA terminator (obtained from P-L Biochemicals) was ligated into pUC18 which had been cut with Sst I and filled in using T4 DNA polymerase to form plasmid pDR813. The 28 bp terminator fragment was similarly inserted into the filled-in Cla I site of pIC19R to construct plasmid pDR812. Plasmid pDR540 (Russell et al., Gene 20: 231-243, 1982), which contains the TAC promoter and lac operator (lacO), was cut with Hind III, filled in with Klenow polymerase, and digested with Bam HI. The resulting ~92 bp fragment, comprising the TAC promoter and lacO, was ligated into Bam HI + Sma I - cut pDR813 to form pDR814. Plasmid pDR814 was then digested with Eco RI and Hind III, and the fragment comprising the trpA terminator, TAC promoter, and lacO was isolated and ligated into PDR812 which had been digested to completion with Hind III and partially digested with Eco RI. The resultant plasmid was designated pDR815.

Plasmid Zem99 (see Example 1) was digested with Bam HI and Xba I, and the fragment comprising the t-PA cDNA (including the pre-pro sequence) was isolated. Plasmid pDR815 was cut with Bam HI and Xba I and ligated to the t-PA fragment to construct plasmid pDR816.

Ten μg of pDR816 were digested to completion with five units of Bgl II and the ends filled in using DNA polymerase I (Klenow fragment). The DNA was then digested to completion with five units of Xba I, extracted with phenol-$CHCl_3$, ethanol precipitated, and electrophoresed on a 0.7% agarose gel. A 3050 bp fragment (comprising pIC19R, TAC promoter, and t-PA pre-pro sequences) was eluted from the gel.

The 3050 bp fragment and the randomly deleted t-PA cDNA sequences were ligated overnight at 16°C. Ligation mixtures were used to transform competent E. coli JM83 cells. Transformed cells were plated on ampicillin and resistant colonies were selected and transferred to fresh ampicillin-containing plates in triplicate. The plates were incubated overnight at 37°C, and colonies were transferred to nitrocellulose filters. The filters were placed on ampicillin plates and incubated four hours at 37°C, then treated with $CHCl_3$ vapor for 10 minutes. The filters were air-dried for 10 minutes and placed directly onto a fibrin plate and incubated at 37°C overnight. The filters were lifted off the plates, and colonies capable of causing fibrin lysis were picked for further analysis. A duplicate set of filters was similarly processed for colony immuno blot assay. Colonies capable of producing t-PA-like polypeptide were identified.

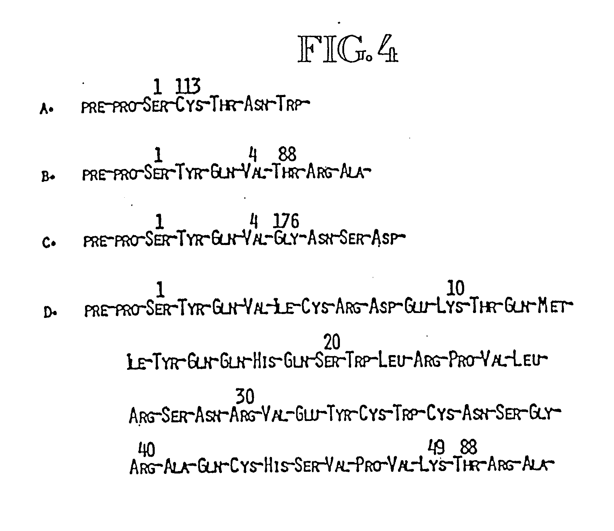

One plasmid, designated pDR817, was characterized in detail. The plasmid was digested with Bam HI and Xba I, and a 1270 bp t-PA fragment was gel-purified and subcloned into M13mp18 (replicative form). The DNA sequence of the insert was determined by the dideoxy method. Results indicated that pDR817 lacked nucleotides 192-524 (numbering based on that of Pennica et al., ibid.). It thus encodes a mature protein consisting of 416 amino acids with an amino-terminus, as shown in Figure 4A. Amino acids 2-112 of naturally-occurring t-PA were deleted.

## C. Mammalian Cell Expression Vectors

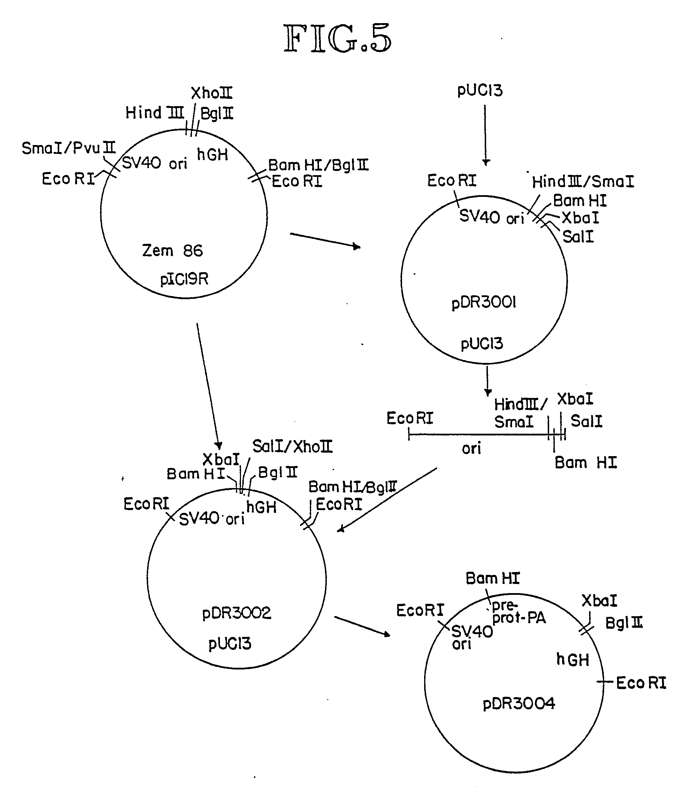

Vectors comprising additional deletion mutant sequences were constructed in the following manner. Plasmid Zem86 (described in Example 1) was digested with Hind III and the ends filled in using DNA polymerase I (Klenow fragment). The linearized DNA was then digested with Eco RI, and a ~350 bp fragment, comprising the SV40 ori sequence, was gel-purified and ligated to Sma I + Eco RI digested pUC13. The resultant vector was designated pDR3001. plasmid pDR3001 was digested with Sal I and Eco RI and the ~350 bp fragment, comprising SV40 ori and polylinker sequences, was gel-purified. Zem86 was partially digested with Eco RI and completely digested with Xho I to remove the SV40 ori sequence. The SV40 fragment from pDR3001 was then joined to the linearized Zem86. The resultant plasmid was designated pDR3002 (Figure 5).

Mammalian cell expression vectors were then constructed. Plasmid pDR3002 was digested with Bam HI and Xba I. The bacterial vectors described in Example 3B were digested with Bam HI and Xba I, and the t-PA sequences were gel-purified and ligated to the linearized pDR3002. The resultant vectors (Table 1) contain expression units of SV40 promoter - mutant t-PA sequence - hGH terminator. The vector pDR3004 (Figure 5) comprises the mutant t-PA sequence from pDR817. The primary translation product from pDR3004 is predicted to have the junction sequence shown in Figure 4A. E. coli LM1035 transformed with pDR3004 has been deposited with American Type Culture Collection under Accession No. 53445.

## TABLE 1

### Summary of Sequential Deletion Mutants

| Plasmid | Sequence at Pre-Pro Junction | Domains* |
|---|---|---|
| pDR3004 | AGA TCG TGC ACC AAC<br>1 113 114 115<br>Arg Ser Cys Thr Asn | $K_1$ (partial), $K_2$, SP |
| pDR3006 | AGA TCC CTG GGG AAC<br>1 138 139 140<br>Arg Ser Leu Gly Asn | $K_1$ (partial), $K_2$, SP |
| pDR3007 | AGA TCC ACC AAC AGT<br>1 114 115 116<br>Arg Ser Thr Asn Trp | $K_1$ (partial), $K_2$, SP |
| pDR3008 | AGA TCG GGA AAC AGT<br>1 176 177 178<br>Arg Ser Gly Asn Ser | $K_2$, SP |
| pDR3010 | AGA TCG GGT GCC TCC<br>1 198 199 200<br>Arg Ser Gly Ala Ser | $K_2$ (partial), SP |
| pDR3011 | AGA TCT GAG GGA ACC<br>1 175 176 177<br>Arg Ser Glu Gly Asn | $K_2$, SP |
| pDR3012 | AGA TCG AAG TAC AGC<br>1 162 163 164<br>Arg Ser Lys Tyr Ser | $K_1$ (partial); $K_2$, SP |
| pDR3013 | AGA TCC TGC CAG CAG<br>1 62 63 64<br>Arg Ser Cys Gln Gln | GF (partial), $K_1$,<br>$K_2$, SP |
| pDR3014 | AGA TCG AAT GGG TCA<br>1 184 185 186<br>Arg Ser Asn Gly Ser | $K_2$, SP |
| pDR3016 | AGA TCG GGC ACC TGC<br>1 60 61 62<br>Arg Ser Gly Thr Cys | GF (partial), $K_1$,<br>$K_2$, SP |

* GF – growth factor, $K_1$ – Kringle 1, $K_2$ – Kringle 2,
SP – serine protease

### Example 3 - Replacement of the Finger Domain with Consensus Finger Sequences

Replacement of the t-PA finger domain with a consensus finger region results in the elimination of potential proteolytic cleavage sites at Arg-27, Lys-49, and Arg-89. Eight finger replacement sequences were constructed, based on an analysis of the finger domains of fibronectin and t-PA.

The consensus finger sequences were constructed from oligonucleotides as described below, then inserted into the t-PA coding sequence. To facilitate this insertion, Kpn I sites were introduced at two locations downstream (3') of the region encoding the wild-type finger domain. Digestion of the resulting sequences with Bgl II and Kpn I resulted in the deletion of the finger domain or the finger, growth factor, and Kringle 1 domains. In a similar manner, a growth factor-less molecule may be generated by introducing a Kpn I site between the growth factor and Kringle 1 sequences.

## A. Kpn I Site Insertion Between the Finger and Growth Factor Domains

In order to place a Kpn I site after the finger domain in t-PA, a mutagenesis was performed with oligonucleotide ZC986 (TTT GAC AGG TAC CGA GTG GCA). A supernatant of a phage M13 clone containing the 5' Bam HI-Eco RI fragment of the t-PA cDNA was prepared. 100 µl of this supernatant was used to infect E. coli RZ1032 in 100 µl of YT medium supplemented with 0.1 µg/ml uridine. This culture was incubated at 37° C, with vigorous shaking, overnight. Growing the M13 in RZ1032 produces phage containing uridine which are viable in RZ1032 but not in JMI01.

The cells were spun out, and the phage supernatant was used to reinfect E. coli RZ1032. This second passage was performed to dilute out any JM101-derived phage which contained no uracil. Again, the cells were spun out and the phage were plated on JM101 and RZ1032. Normal viability was observed on RZ1032 plates (indicating phage at $10^9$ pfu/ml), but no plaques were observed on JM101 cells. A complementary strand primed with the mutagenic oligonucleotide was then produced in vitro. The new strand, contain ing the mutation, contained thymidine and was therefore viable in JIM101; the wild-type template was not.

Template DNA was prepared by PEG precipitation of the phage supernatant followed by phenol-chloroform extraction and ethanol precipitation. One µg of this template DNA was hybridized with 10 g of oligonucleotide ZC986 by briefly boiling, incubating at 65° C for 5 minutes, and then slowly bringing the temperature down to 4° C before adding 10 µl 0.2 M HEPES pH 7.8, 2 µl 100 mM DTT, 1 µl 1 M MgCl₂, 20 µl 2.5 mM each dNTP, 10 µl 10 mM ATP, 1 µl 2.5 U/µl Klenow, and 2 µl 1 U/µl T₄ DNA ligase, final volume adjusted to 100 µl with H₂O. After extension at 37° C for 2 hours, the DNA was transfected into competent JM101 cells. A control extension (minus oligonucleotide) was performed to compare the amount of background produced by extension by priming on contaminating RNA or DNA species. The transfection produced zero plaques with unmutagenized template, 150 on control extension (minus oligonucleotide) and 300 with mutagenized template.

The plates were screened by hybridizing a plaque lift with $^{32}$p-labeled mutagenic oligonucleotide and washing in 3 M TMAC1 (Wood et al., Proc. Natl. Acad. Sci. USA 82: 1585-1588, 1985) at Tm-5° C for 30 minutes and also by sequencing randomly picked plaques. One positive clone was obtained.

## B. Kpn I Site Insertion Between the Two Kringle Domains

In order to produce a mutant t-PA which lacks the growth factor and Kringle 1 domains, and which will be readily converted to a finger-replacement mutant, another Kpn I site was inserted between the Kringle 1 and Kringle 2 domains. This was accomplished by mutagenesis of the ZC986-mutagenized template (described above) with oligonucleotide ZC1133 (ACT GTT TCC CTC AGA AAC TGG TAC CGA GCA GAA CTC TGA GCT). This oligonucleotide loops in sequences which, once cut with Kpn I and re-ligated, form a finger/ Kringle 2 junction with the sequence: Val Pro Val/Ser Glu Gly.

The mutagenesis was performed as in section A above. The resulting doubly mutagenized material was prepared as replicative form (RF) DNA, cut with Kpn I, re-ligated, and sequenced, thus producing a DNA sequence encoding a t-PA homolog lacking growth factor and Kringle 1 domains.

C. Production of Finger Replacement Domains

The finger region replacements shown in Table 2 were constructed.

## TABLE 2

| Finger | Encoded Amino Acid Sequence | Oligonucleotides* |
|---|---|---|
| t-PA wild-type: | CRDEKTQMIYQQHQSWLRPVLR-SNRVEYCWC--N-SGRAQC | |
| Consensus 1: | CFD--NGKSYKIGETWERPYE--GFMLS-CTCLGNGRGEFRC | (ABC) |
| Consensus 2: | CHDEKTGSSYKIGEQWERPYL-SGNRLE-CTCLGNGSGRWQC | (DEF) |
| Consensus 3: | CFD--NGKSYKIGETWERPYE--GFMLS-CTCLGNGSGRWQC | (ABF) |
| Consensus 4: | CFD--NGKSYKIGEQWERPYL-SGNRLE-CTCLGNGRGEFRC | (AEC) |
| Consensus 5: | CFD--NGKSYKIGEQWERPYL-SGNRLE-CTCLGNGSGRWQC | (AEF) |
| Consensus 6: | CHDEKTGSSYKIGETWERPYE--GFMLS-CTCLGNGSGRWQC | (DBF) |
| Consensus 7: | CHDEKTGSSYKIGEQWERPYL-SGNRLE-CTCLGNGRGEFRC | (DEC) |
| Consensus 8: | CHDEKTGSSYKIGETWERPYE--GFMLS-CTCLGNGRGEFRC | (DBC) |

*A = ZC1116/1117   D = ZC1122/1123
 B = ZC1118/1119   E = ZC1124/1125
 C = ZC1120/1121   F = ZC1126/1127

TABLE 3

ZC1116
GAT CTT ATC AAG TCA TAT GTT TTG ATA ATG GAA AAT CTT ATA A

ZC1117
CTC CAA TTT TAT AAG ATT TTC CAT TAT CAA AAC ATA TGA CTT GAT
AA

ZC1118
AAT TGG AGA AAC ATG GGA ACG GCC GTA TGA AGG ATT TAT GCT TTC
T

ZC1119
CAT GTA CAA GAA AGC ATA AAT CCT TCA TAC GGC CGT TCC CAT GTT
T

ZC1120
TGT ACA TGC CTA GGA AAT GGC CGC GGA GAA TTT AGA TGT CAT TCG
GTA C

ZC1121
CGA ATG ACA TCT AAA TTC TCC GCG GCC ATT TCC TAG G

ZC1122
GAT CTT ATC AAG TCA TAT GTC ATG ATG AAA AAA CAG GCT CGA GTT
ATA A

ZC1123
CTC CAA TTT TAT AAC TCG AGC CTG TTT TTT CAT CAT GAC ATA TGA
CTT GAT AA

ZC1124
AAT TGG AGA ACA ATG GGA ACG GCC GTA TCT TTC TGG AAA TCG ATT
AGA A

ZC1125
CAT GTA CAT TCT AAT CGA TTT CCA GAA AGA TAC GGC CGT TCC CAT
TGT T

ZC1126
TGT ACA TGC CTA GGA AAT GGT TCC GGA AGA TGG CAA TGT CAT TCG
GTA C

Z1127
CGA ATG ACA TTG CCA TCT TCC GGA ACC ATT TCC TAG G

The eight consensus sequences were generated from the indicated oligonucleotides. The oligonucleotides (Table 3) were produced using an Applied Biosystems Model 380A DNA synthesizer. First, the twelve oligonucleotides were kinased and simultaneously labeled to a low specific activity with $\gamma$-$^{32}$P ATP by incubating each with polynucleotide kinase at 37°C for $\frac{1}{2}$ hour. Then the indicated eight combinations (ABC, DEF, ABF, AEC, AEF, DBF, DEC and DBC) were produced by mixing the appropriate oligonucleotides, adding DNA ligase, and incubating at 37°C for 1 hour. The products of this reaction were sorted out on a 6% polyacrylamide-8 M urea sequencing gel. The bands corresponding to the DNA coding for full-length finger domains were cut out, and the DNA was eluted in 2.5 M ammonium acetate. The DNA was ethanol-precipitated and resuspended in water to a concentration of 1 pmole/$\mu$l.

RF DNA was prepared from the positive clone described in Example 3A, and the Bam HI to Eco RI t-PA

17

fragment was purified. Plasmid Zem219a (described in Example 10A) was digested with Xba I and then partially digested with Eco RI. The 1010 bp fragment, containing the 3' t-PA coding region, was purified. Plasmid Zem219b (described in Example 10A) was digested with Bam HI and Xba I and ligated to the 5' t-PA fragment (Bam HI-Eco RI) and the 1010 bp Eco RI-Xba I fragment. The resulting vector, designated Zem238, contains a Kpn I site after the finger domain. Zem238 was digested with Bgl II and Kpn I, gel purified to remove the wild-type finger domain, and ligated with each of the eight consensus sequences to generate expression vectors 238-Fcon 1 to 238-Fcon 8.

In a similar manner, vectors encoding a t-PA homolog lacking the GF and K1 domains and containing a consensus finger domain were constructed. RF DNA was prepared from the mutagenized clone described in Example 3B, and the 5' Bam HI to Eco RI fragment was purified. This fragment was ligated to the 1010 bp Eco RI-Xba I fragment from Zem219a and the Bam HI-Xba I digested Zem219b. The resul tant vector, designated Zem248, contains a Kpn site at the beginning of the K2 domain. Plasmid Zem248 was digested with Bgl II and Kpn I, gel purified fo remove the wild-type finger domain, and ligated with each of the eight consensus sequences to generate expression vectors 248-Fcon 1 to 248-Fcon 8.

## Example 4 - Deletion of Finger and Growth Factor Sequences

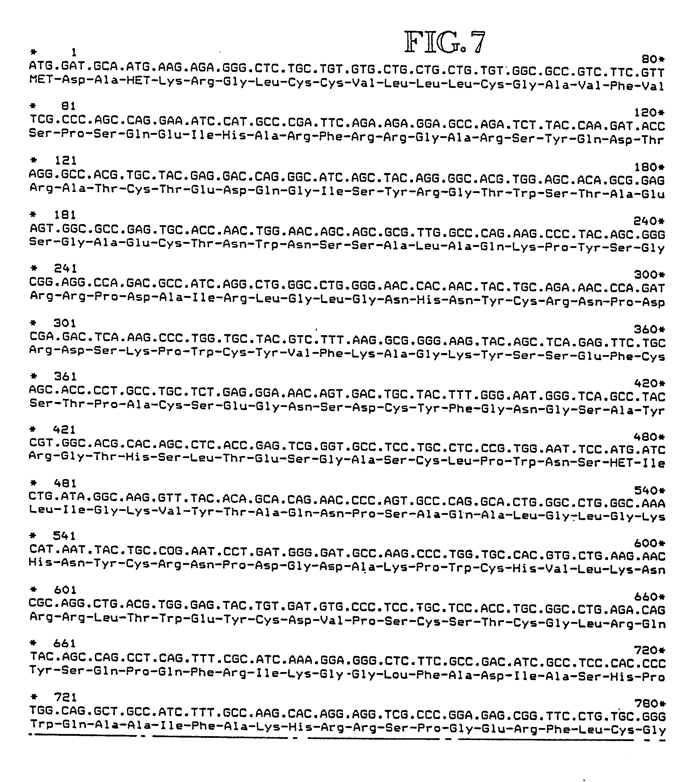

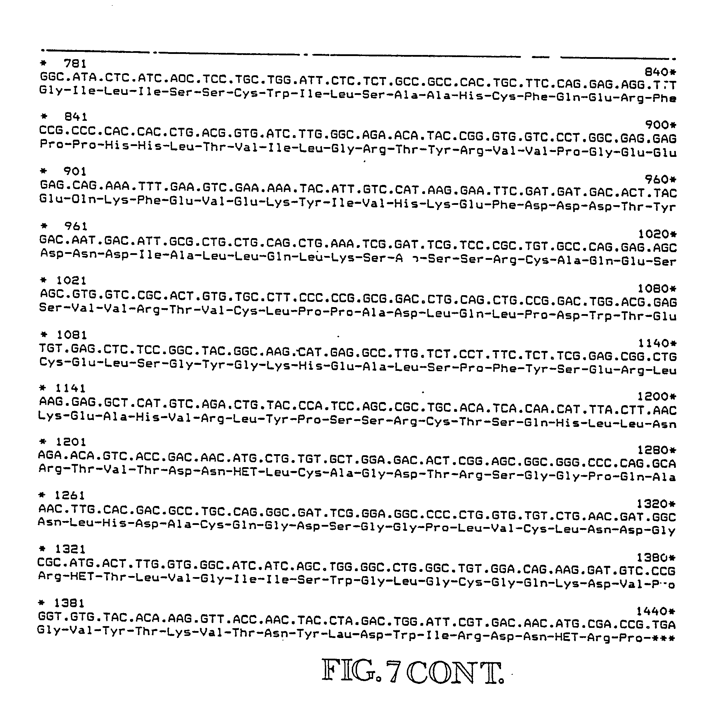

The strategy for deleting the finger and growth factor sequences used as starting material the plasmid pDR1496, which comprises the t-PA coding sequence. S. cerevisiae strain E.8-11C transformed with pDR1496 has been deposited with the American Type Culture Collection under Accession No. 20728. To prepare a template for mutagenesis of the t-PA sequence, 1 μg of pDR1496 was digested with 5 units each of Sph I and Xba I for 2 hours at 37°C. The DNA was electrophoresed on a 0.7% agarose gel and a fragment of ~2100 bp was purified. This fragment was ligated to Sph I + Xba I digested M13tg130 (replicative form; obtained from Amersham; Kieny et al., Gene 26: 91, 1983) to construct M13tg130W. The recombinant phage were transfected into E. coli JM103 and single-stranded template DNA was prepared. Oligonucleotide-directed deletion mutagenesis was carried out, using 20 pmoles phosphorylated mutagenic primer (sequence: 5' CGT GGC CCT GGT ATC TTG GTA AG3' ) and 1 pmole template DNA in 20 mM Tris pH 7.5, 10 mM MgCl2, 50 mM NaCl, 1 mM DTT at 65°C for 10 minutes. The mixture was then incubated for 5 minutes at room temperature and placed on ice. Ten μl of 20 mM Tris pH 7.5, 10 mM MgCl2, 2 mM ATP, 10 mM DTT containing 1 mM dNTPS, 2.5 units Klenow fragment and 3.5 units T4 DNA ligase were added and the annealed DNA mixture was incubated for 3 hours at 15°C. The DNA was transfected into E. coli JM103, and the cells were plated on YT agar and incubated at 37°C. Plaques were screened to identify mutant plaques. A mutant sequence having the desired deletion of finger and growth factor sequences was designated clone #2600. The sequence of the encoded protein, designated 2600, is shown in Figure 7.

## Example 5 - Deletion of Finger, Growth Factor and Kringle 1 Coding Sequences

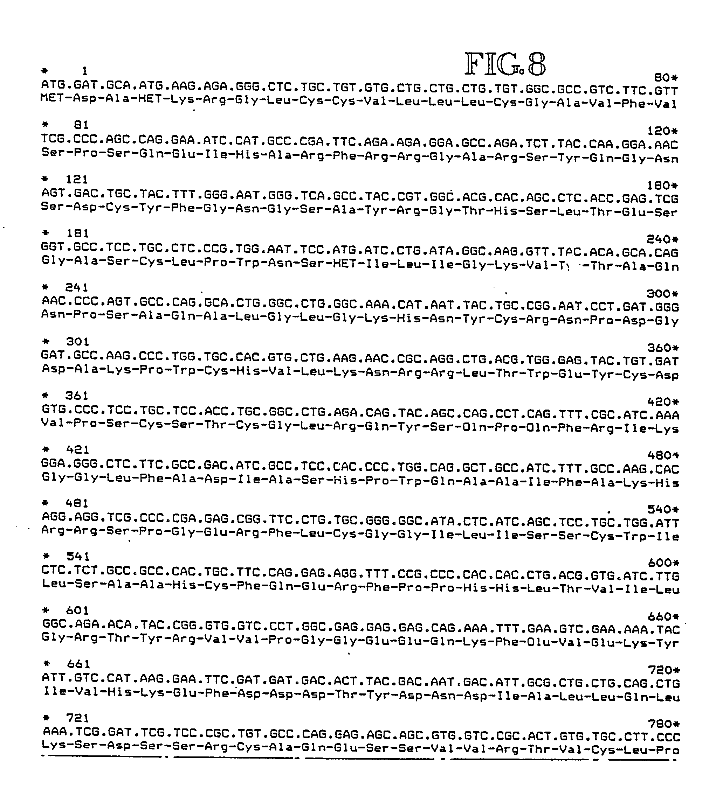

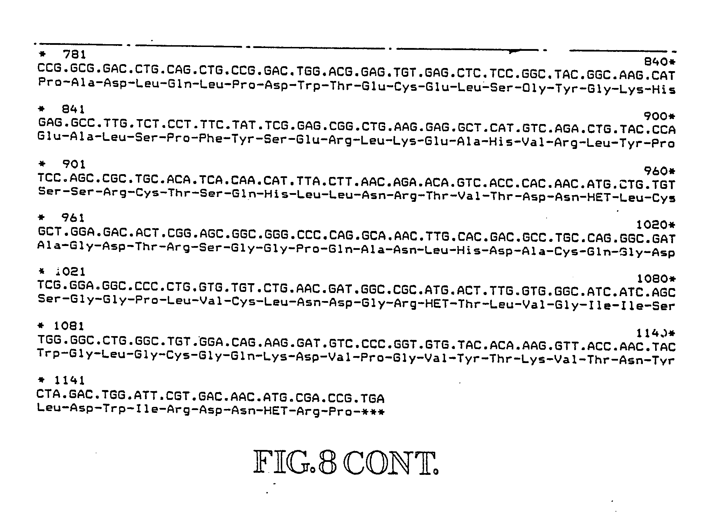

The finger, growth factor, and Kringle 1 sequences were deleted in a second mutagenesis procedure using the single-stranded M13tg130W template described in Example 4. Mutagenesis was carried out as described in Example 4 using an oligonucleotide primer having the sequence 5' GCA GTC ACT GTT TCC TTG GTA AG3' . Mutant plaques were screened as previously described. A clone having the correct deletion was designated #2700. The sequence of the encoded protein, designated 2700, is shown in Figure 8.

## Example 6 - Elimination of the Carbohydrate Addition Site(s) in t-PA

Tissue plasminogen activator contains four potential glycosylation sites (amino acid sequence Asn-X-Ser/Thr). According to Pohl et al. (Biochemistry 23: 3701-3707, 1984), three of these sites (Asn-117, -184 and -448) are glycosylated in t-PA obtained from Bowes melanoma cells. By altering the Asn residues at the glycosylation sites to other amino acid residues, the glycosylation is blocked. It is particularly preferred that the Asn residues be changed to Gln residues. Alterations in the DNA sequence encoding t-PA are made by site-specific mutagenesis. Nucleotide changes may be made singly or in combination to alter one or more of the glycosylation sites. Additionally, mutated sequences may be combined with DNA fragments comprising other deletions, insertions or substitutions of the t-PA coding sequence in order to generate new proteins comprising several mutations in combination.

Site-specific mutagenesis was carried out on single-stranded M13 phage templates using mutagenic primers ZC294, ZC295 and ZC297 (see Table 4) to produce the alterations at Asn-117, -184 and -448, respectively. DNA sequences encoding mutant t-PAs were then constructed comprising single, double or triple mutations by replacing the t-PA coding sequences in pDR1296. The following plasmids were constructed: (1) pDR1601, comprising the mutation at Asn-117; (2) pDR1602, comprising the mutation at Asn-184; (3) pDR1603, comprising the mutation at Asn-448; (4) pDR1604, comprising the mutations at Asn-117 and -448; (5) pDR1605, comprising the mutations at Asn-184 and -448; and (6) pDR1606, comprising the three mutations. All pDR1600 series plasmids were digested with Bgl II and Xba I restriction enzymes, the t-PA fragments purified and ligated into the mammalian expression vector Zem94, which had been pre-cut with Bgl II and Xba I and purified away from the wild-type t-PA coding sequences. The resultant 1600 series mutant expression plasmids were then cotransfected into mammalian cells, and after selection with methotrexate, high producer clones were isolated and the proteins, designated 1601 through 1606, were purified and characterized by fibrin binding, clot lysis and plasma half-life studies.

## TABLE 4

| Primer | Sequence |
|---|---|
| ZC294 | 5'ACC AAC TGG CAA TCT TCT GCG TTG GCC3' |
| ZC295 | 5'TGC TAC TTT GGT CAA GGG TCA GCC3' |
| ZC297 | 5'CAA CAT TTA TTG CAA AGA ACA GTC3' |

### Example 7 - Deletion of Growth Factor Coding Sequence

A mutant sequence lacking the growth factor domain coding sequence was constructed by deletion mutagenesis using oligonucleotide ZC820 ($^5$ GTA GCA CGT GGC CCT GGT TTT GAC AGG CAC TGA GTG$^3$ ) and an M13 phage template containing the 5' Bam HI-Eco RI t-PA fragment. This deletion joined the codons for amino acids 49 and 88 of mature t-PA (Figure 4D). A correct clone was identified by sequencing; however, the Bam HI site had been destroyed so the RF DNA was digested with Pvu II and Hind III. A 713 bp fragment comprising the mutated t-PA sequence was isolated and ligated to Sma I and Hind III - cut pUC18. The ligation mixture was transformed into E. coli HB101. A correct clone was identified and designated pUC18-820.

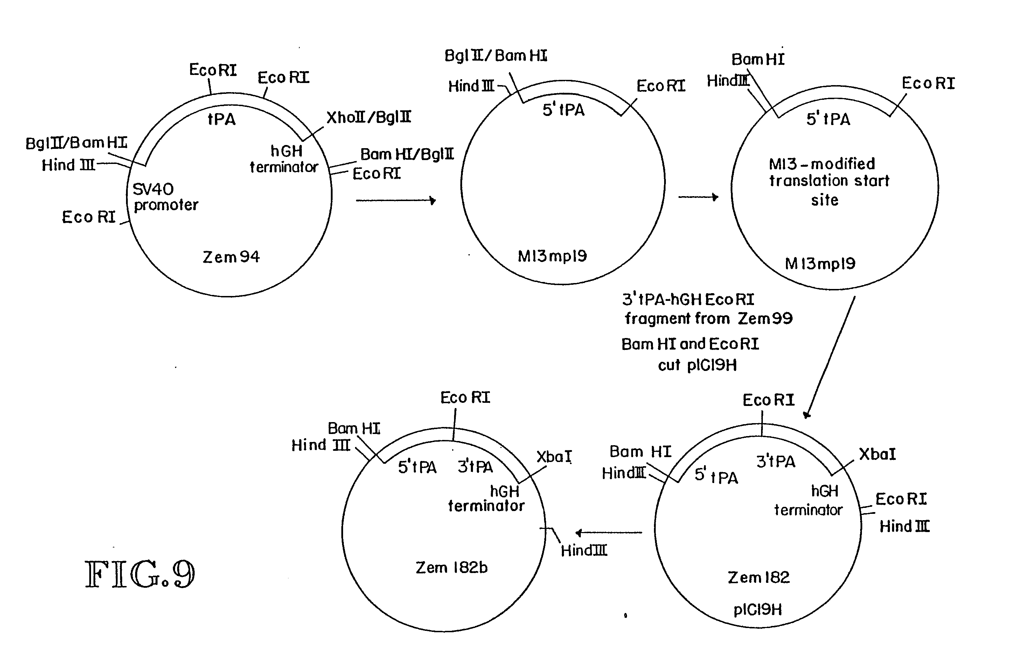

Figure 9 depicts the construction of a vector used to shuttle modified t-PA fragments prior to insertion into an expression vector. The sequence just upstream of the ATG start codon of the t-PA sequence in Zem94 was altered by site-specific mutagenesis, resulting in the positioning of Hind III and Bam HI sites adjacent to the ATG. The resultant nucleotide sequence contains an adenine in the -3 position. Single-stranded M13 template DNA was prepared by inserting a ~800 bp Hind III-Eco RI fragment from Zem94 comprising polylinker, pre-pro, and a portion of the mature t-PA sequences into M13mp19. Site-specific mutagenesis was carried out essentially as described by Zoller et al. (Manual for Advanced Techniques in Molecular Cloning Course, Cold Spring Harbor Laboratory, 1983), using the oligonucleotide ZC444 ($^5$ CAT CCA TGG ,TGG ATC CAA GCT TGG C$^3$ ) as mutagenic primer. Oligonucleotide ZC87 ($^5$ TCC CAG TCA CGA CGT$^3$ ) was used as second primer. The mutated inserts were sequenced by the dideoxy method, and a clone in which polylinker sequences had been deleted and the Bam HI site at the 5' end of the pre-pro sequence had been restored was selected. This phage clone was digested with Bam HI and Eco RI, and the 5' t-PA sequence was isolated. Zem99 was digested with Eco RI, and the fragment comprising the 3' portion of the t-PA sequence and the hGH terminator was isolated. The two fragments were then joined with Bam HI + Eco RI digested pIC19H (Marsh et al., Gene 32: 481 486, 1984) in a three-part ligation. A plasmid containing the t-PA fragments in the proper orientation was selected and designated Zem182. Plasmid Zem182 was partially digested with Eco RI, and the ends were filled using DNA polymerase I (Klenow fragment). The linearized plasmid was gel-purified and recircularized using T₄ DNA ligase. A plasmid in which the Eco RI site at the 3' end of the hGH terminator was destroyed was selected and designated Zem182b (Figure 9).

Plasmid pUC18-820 was digested with Bgl II and Eco RI, and the modified t-PA sequence was purified.

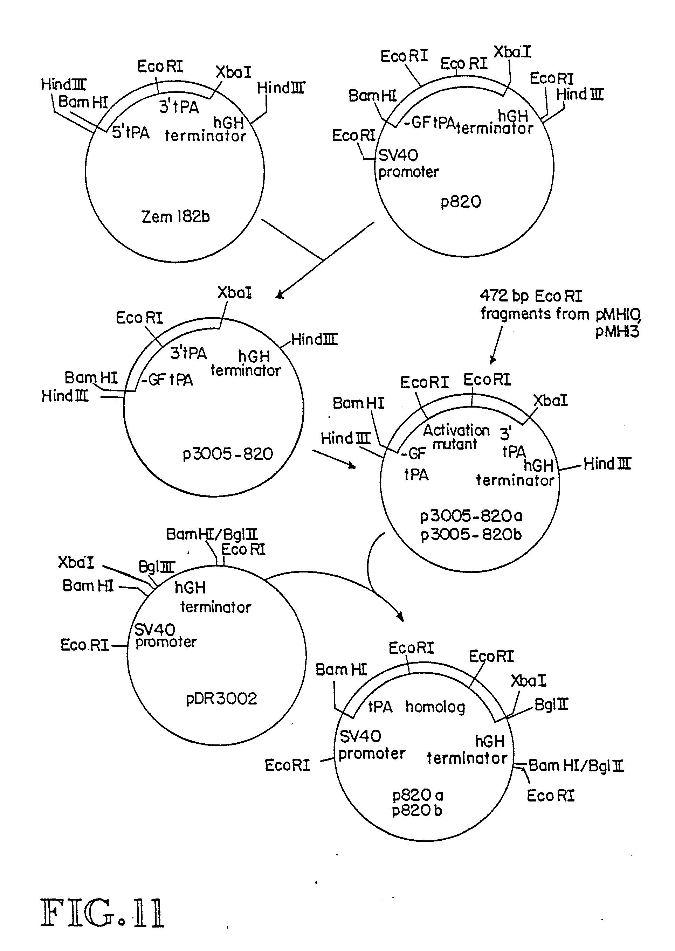

This fragment was ligated together with the 472 by Eco RI internal t-PA fragment from Zem99 and with purified Zem182b vector DNA after digestion with Bgl II and Eco RI to remove wild-type t-PA sequences. The resultant plasmid was digested with Bam HI and Xba I and inserted into vector pDR3002 (Example 2C and Figure 5) to make plasmid p820 (Figure 11). The protein encoded by this vector was designated 820.

## Example 8 - Site-Specific Mutagenesis of the Activation Site

For site-specific mutagenesis, a 472 bp Eco RI fragment comprising the t-PA sequence from bp 802 to bp 1274 was isolated from Zem99 and cloned into the Eco RI site of M13mp18 (replicative form). The recombinant phage were transfected into E. coli (JM101), and anti-sense strand DNA was isolated.

Site-specific mutagenesis was then carried out on the single-stranded anti-sense template DNA using one of the mutagenic primers shown in Table 5 and ZC87 as second primer. Oligonucleotides ZC487, 488, 489 and 620 change the Phe at position 274 to Glu, Gly, Arg or Pro, respectively. Oligonucleotides ZC797, 874, 1013 and 1027 change the Arg at position 275 to Gly, Leu, Pro or Asp, respectively. Oligonucleotide 621 introduces a Leu in place of the Lys at position 277. Oligonucleotide 928 changes the Ile at position 276 to Pro. Oligonucleotide 875 changes Arg (275) to Leu and oligonucleotide 927 changes Phe (274) to Pro in the mutant which previously had Lys (277) converted to Leu. Thus, oligonucleotides 875 and 927 can be used to generate double mutations. Twenty pmoles of phosphorylated mutagenic primer and 20 pmoles of the second primer were combined with one pmole of single-stranded template in 10 $\mu$l of 20 mM Tris pH 7.5, 10 mM MgCl$_2$, 50 mM NaCl, 1 mM DTT and incubated at 65°C for 10 minutes, then 5 minutes at room temperature, and placed on ice. Ten $\mu$l of 20 mM Tris pH 7.5, 10 mM MgCl$_2$, 2 mM ATP, 10 mM DTT containing 1 mM dNTPS, 2.5 units Klenow polymerase, and 3.5 units DNA ligase were added to the annealed DNA, and the mixture was incubated 3 hours at 15°C. The DNA was then transfected into competent E. coli JM101, and the cells were plated on YT agar and incubated at 37°C. The DNA was then transferred to nitrocellulose and prehybridized at the Tm-4°C of the mutagenic primer for 1 hour in 6x SSC, 10x Denhardt's and hybridized to $^{32}$P-labeled mutagenic primer at Tm-4°C in the same solution. After three washes at Tm-4°C, filters were exposed to X-ray film overnight. Additional wash steps were performed at 5°C higher increments as necessary to identify mutant plaques. The mutated inserts were sequenced by the dideoxy method.

## TABLE 5

ZC463 5'CCT CAG TTT AAA ATC AAA3'

ZC487 5'CAG CCT CAG GAG CGC ATC AAA3'

ZC488 5'CAG CCT CAA GGT CGC ATC AAA3'

ZC489 5'CAG CCT CAG AGA CGC ATC AAA3'

ZC620 5'CAG CCT CAG CCT CGC ATC AA3'

ZC621 5'TTT CGC ATC CTC GGA GGG CTC3'

ZC797 5'CTT CAG TTC GGC ATC AAA3'

ZC814 5'G CCT CAG TTC GGC ATC AAA GG3'

ZC874 5'CT CAG TTT CTC ATC AAA GG3'

ZC875 5'CT CAG TTT CTC ATC CTC GG3'

ZC927 5'CAG CCT CAG CCT CGC ATC CT3'

ZC928 5'CAG TTT CGC CCC AAA GGA GG3'

ZC1013 5'CT CAG TTT CCC ATC AAA GG3'

ZC1027 5'CCT CAG TTT GAC ATC AAA GG3'

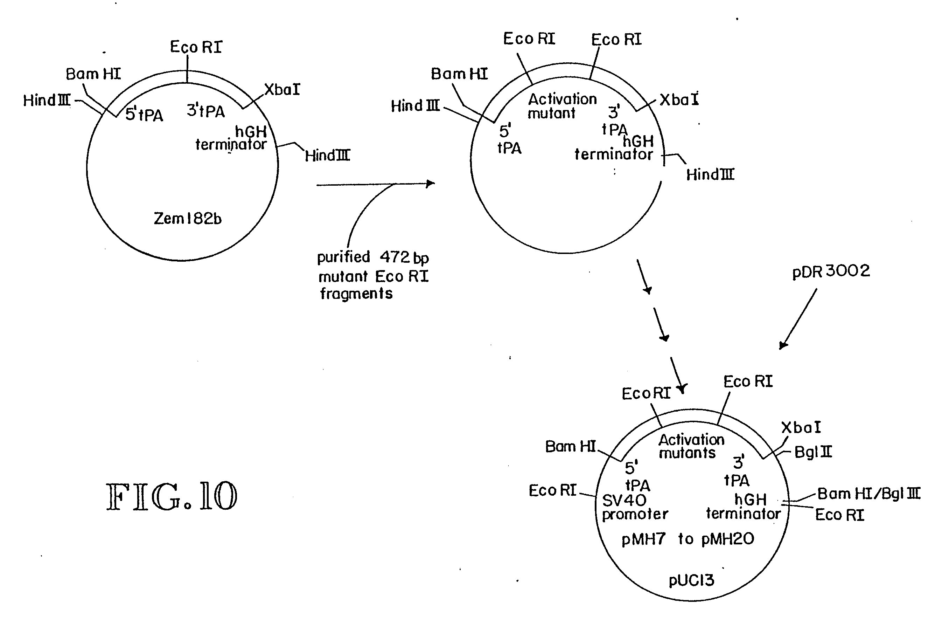

Expression vectors for the altered sequences were then constructed (Figure 10). Replicative form (RF) DNA was prepared from the mutagenized phage, and the modified t-PA sequences were purified as Eco RI fragments. Plasmid Zem182b was digested with Eco RI, the vector sequences containing the 5′ and 3′ portions of the t-PA coding sequence were treated with calf alkaline phosphatase, and the modified t-PA sequences were inserted. The resultant plasmids were digested with Bam HI and Xba I, and the t-PA fragments were inserted into Bam HI + Xba I - cut pDR3002. The resultant vectors were designated pMH7 through pMH2O (Table 6).

## TABLE 6

| Protein | Sequence of Amino Acids 273-279 |
|---|---|
| Native t-PA | Gln-Phe-Arg-Ile-Lys-Gly-Gly |
| pMH7 | Gln-Gly-Arg-Ile-Lys-Gly-Gly |
| pMH8 | Gln-Phe-Arg-Ile-Leu-Gly-Gly |
| pMH9 | Gln-Arg-Arg-Ile-Lys-Gly-Gly |
| pMH10 | Gln-Pro-Arg-Ile-Lys-Gly-Gly |
| pMH11 | Gln-Glu-Arg-Ile-Lys-Gly-Gly |
| pMH12 | Gln-Phe-Lys-Ile-Lys-Gly-Gly |
| pMH13 | Gln-Phe-Gly-Ile-Lys-Gly-Gly |
| pMH14 | Gln-Pro-Arg-Ile-Leu-Gly-Gly |
| pMH15 | Gln-Phe-Leu-Ile-Lys-Gly-Gly |
| pMH16 | Gln-Phe-Leu-Ile-Leu-Gly-Gly |
| pMH17 | Gln-Phe-Arg-Pro-Lys-Gly-Gly |
| pMH18 | Gln-Phe-Pro-Ile-Lys-Gly-Gly |
| pMH19 | Gln-Phe-Asp-Ile-Lys-Gly-Gly |
| pMH20 | Gln-Phe-Gly-Ile-Leu-Gly-Gly |

A second construct encoding t-PA with the Pro substitution at amino acid 276 was made as follows. M13tg130W template (Example 4) was annealed to a mutagenic oligonucleotide ($^5$ GAG CCC TCC CTT TGG GCG GAA CTG AGG C$^3$ ). Mutagenesis was carried out essentially as described in Example 4. Mutant plaques were screened and sequenced to identify clones having the desired mutation. One such clone and its encoded protein were designated #2550.

## Example 9 - Combination of Domain and Active Site Mutations and Expression Vector Construction

### A. Mutants with Deletion of Growth Factor Domain

Two mutant sequences were constructed which combined the minus growth factor fibrin binding domain with the Phe (274) to Pro or Arg (275) to Gly mutations (Figure 11). Plasmid p820 (Example 7), was digested with Bam HI and Eco RI. The fragment comprising the t-PA -GF sequence was purified and ligated to Bam HI + Eco RI digested Zem182b to construct p3005-820. This plasmid was digested with Eco RI, and the 472 bp mutant t-PA fragments were individually inserted into the Eco RI site of p3005-820. The resulting derivatives of pMH10 and pMH13 were designated p3005-820a and p3005-820b, respectively. The t-PA coding sequences were removed from p3005-820a and p3005-820b as Bam HI-Xba I fragments and were joined to Bam HI + Xba I- cut pDR3002 to construct p820a and p820b, respectively (Figure 11).

### B. Mutants with Deletion of Finger and Growth Factor Domains

Clone #2600 (Example 4) was cut with Bam HI and Eco RI, and the fragment comprising the mutant (-F, -GF) t-PA sequence was purified. This fragment was inserted into Bam HI + Eco RI - cut Zem182b to construct p3005-2600. Plasmid p3005-2600 was digested with Eco RI, and the Eco RI fragments from pMH10 and pMH13 (Example 8) were each inserted into the Eco RI site of pDR3005-2600 to construct plasmids p3005-2600a and p3005-2600b, respectively. These plasmids were digested with Bam HI and Xba I, and the t-PA fragments were ligated to Bam HI + Xba I - cut pDR3002 to construct p2600a and p2600b.

Clone #2600 was digested with Sca I and Apa I, and the ~630 bp fragment was purified. The domain-deleted t-PA sequence described in Example 4 was removed from the M13 replicative form with Bgl II and Sca I. The Bgl II + Sca I fragment with the domain-deleted sequence and the Sca I + Apa I fragment with the amino acid substitution mutation were ligated with the Bgl II + Apa I vector fragment from Zem99. The resultant plasmid was designated Zem99-2625 and the encoded protein was designated 2625 (the finger and growth factor domain deletion combined with the Pro (pMH17 or 2550) amino acid substitution).

### C. Mutants with Deletion of Finger, Growth Factor and Kringle 1 Domains

Plasmid p3008 (Example 2) was digested with Bam HI and Eco RI, and a fragment comprising the multiple domain-deleted t-PA sequence was isolated. This fragment was inserted into Bam HI + Eco RI digested Zem182b to construct p3005-3008. Plasmid p3005-3008 was digested with Eco RI, and the Eco RI fragments from pMH10 and pMH13 were inserted into p3005-3008 to construct p3005-3008a and p3005-3008b, respectively. The Bam HI-Xba I t-PA fragments were isolated from p3005-3008a and p3005-3008b and were inserted into Bam HI + Xba I cut pDR3002 to construct p3008a and p3008b, respectively.

Clone pMH17 (Example 8) was digested with Sca I and Apa I, and the ~630 bp fragment was purified. The domain-deleted t-PA sequence described in Example 5 was removed from the M13 (RF) vector with Bgl II and Sca I. The Bgl II-Sca I domain-deletion fragment and the Sca I-Apa I fragment comprising the Pro (276) substitution were ligated to the Bgl II-APa I vector fragment from Zem99. The resultant plasmid was designated Zem99-2725 (the finger, growth factor and Kringle 1 domain deletion combined with the Pro (276) amino acid substitution).

### D. Mutants with the deletion of the growth factor domain in addition to the deletion of the carbohydrate addition site at position 117 in KI and the substitution of Arg to Gly at position 275 or Phe to Pro at position 274

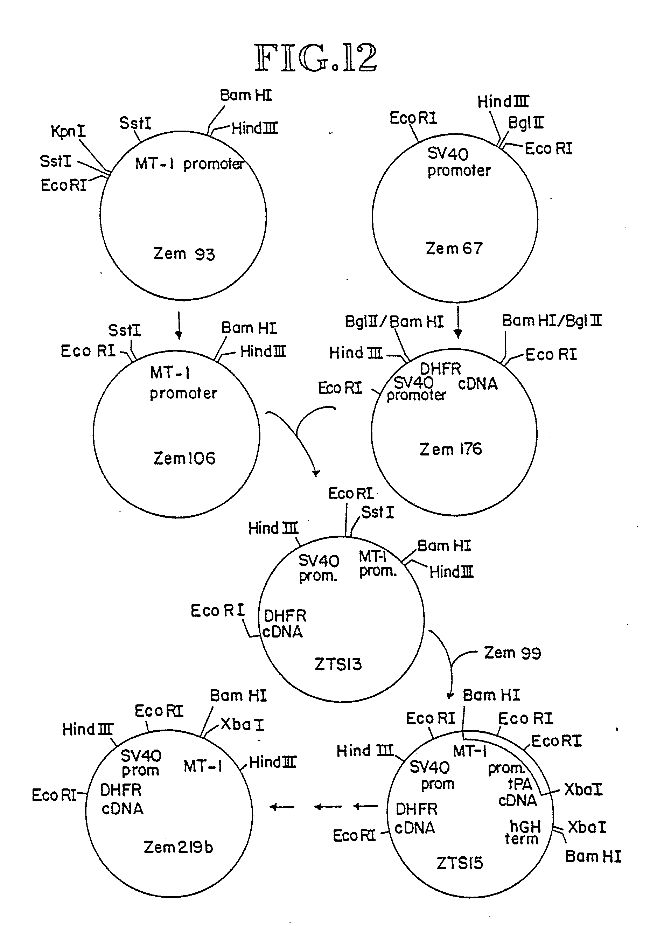

DNA from the t-PA deletion mutant 820 (deleted growth factor domain) was digested with Bam HI and Eco RI. The 614 bp Bam HI-Eco RI DNA fragment was isolated and cloned into the Bam HI and Eco RI sites of M13mp18. Site-directed mutagenesis was performed using the mutagenic primer ZC326 ($^5$ ACCAACTGGCAATCTAGCGCGTTG$^3$ ). Correctly mutated phage clones were identified by dideoxy DNA sequencing. One correctly mutated clone was sequenced throughout the entire insert to ensure integrity of the fragment. RF DNA was prepared from the sequenced clone, digested with Bam HI and Eco RI, and the correct size Bam HI-ECO RI fragment was isolated. The final mammalian expression vector, designated 820C, was constructed by a three-part ligation of Bam HI and Xba I cut Zem219b (see Figure 12 and Example 10A), the 614 bp Bam HI-Eco RI mutated fragment, and the 1010 bp partial Eco RI-Xba I fragment from plasmid pMH13 (encoding the amino acid Gly at position 275). Use of the partial Eco RI-Xba I fragment from pMH10 results in a sequence encoding pro at position 274.

22

**E. Mutants with the deletion of the finger and growth factor domains in addition to the deletion of the carbohydrate addition site at position 117 in KI and the substitution of Arg to Gly at position 275**

DNA from the t-PA deletion mutant 2600 (deleted finger and growth factor domains) was digested with Bam HI and Eco RI. The 473 bp Bam HI-Eco RI DNA fragment was isolated and cloned into the Bam HI and Eco RI sites of M13mp18. Site-directed mutagenesis was performed using standard methods and the mutagenic primer ZC326. Correctly mutated phage clones were identified by dideoxy DNA sequencing. One correctly mutated clone was sequenced throughout the entire insert to ensure integrity of the fragment. RF DNA was prepared from the sequenced clone, and digested with Bam HI and Eco RI. The correct size Bam HI to Eco RI fragment was isolated. The final mammalian expression vector, designated 2600C, was constructed by a three-part ligation of Bam HI and Xba I - cut Zem219b, the 473 bp Bam HI-Eco RI mutated fragment, and the 1010 bp partial Eco RI-Xba I fragment from pMH13 (encoding amino acid Gly at position 275).

## Example 10 - Expression of Fibrinolytic Proteins in Mammalian Cells

### A. Construction of Zem219b

Plasmid pSV2-DHFR (Subramani et al., ibid.) was digested with Cfo I, and the fragment containing the DHFR cDNA and the 3′ attached SV40 sequences was isolated, repaired, and ligated to Bam HI linkers. After digestion with Bam HI, an approximately 800 bp fragment containing the entire cDNA and the SV40 terminator region was purified and ligated to Bam HI-digested pUC8. Zem67 (Example 1) was digested with Bgl II and ligated with the Bam H1 DHFR-SV40 fragment to generate plasmid Zem176. plasmid Zem93 was digested with Sst I and re-ligated to generate plasmid Zem106, in which approximately 600 bp of sequence 5′ to the MT-1 promoter were eliminated. Plasmid Zem106 was digested with Eco RI and ligated to the Eco RI fragment containing the DHFR gene from plasmid Zem176. The resulting plasmid was designated Zts13. Plasmid Zts13 was digested with Bam HI and ligated to the Bam HI fragment from plasmid Zem99 containing the entire t-PA coding region and hGH terminator sequence. The resulting plasmid was designated Zts15. Zts15 was partially digested with Bam HI, repaired, re-ligated and transformed to generate plasmid Zem219, in which the 3′ Bam HI site was destroyed. Plasmid Zem219 was partially digested with Xba I, repaired, re-ligated and transformed to generate plasmid Zem219a, in which the 3′ Xba I site was destroyed. Plasmid Zem219a was digested with Bam HI and Xba I, the vector sequences purified away from the t-PA cDNA sequences, and ligated with an oligomeric Bam HI-Xba I adaptor to generate the expression vector Zem219b (Figure 12), into which mutant Bam HI-Xba I t-PA sequences were inserted.

### B. Expression

DNA sequences encoding fibrinolytic proteins described above were isolated as Bam HI-Xba I fragments and inserted into the vectors Zem94, Zem99, pDR3002 or Zem219b. Expression vectors derived from Zem94, Zem99 and PDR3002 were cotransfected with PSV2-DHFR DNA into Tk⁻BHK cells by the calcium phosphate transfection protocol described earlier. Vectors derived from Zem219b were transfected by the electroporation procedure. Drug selection was applied, and high expressing cell lines were isolated and selected for expansion and scale-up of protein production.

### Example 11 - Purification and Characterization of t-PA Homologs

Mutant proteins were subjected to an initial purification on affinity columns in which the appropriate polyclonal or monoclonal antibody was covalently linked to CNBr-activated Sepharose. Media from transfected BHK cells grown in serum-free media were applied to the column at a rate of 200 ml/hr at 4° C. The column (5 x 1.5 cm) was equilibrated with 0.025 M potassium phosphate buffer, pH 8.5, containing 0.2 M NaCl, 0.2% Tween-80 and 3 mM sodium azide. Serum-free media were applied directly to the column and, following the single passage of the media volume, the column was washed extensively with the same equilibration buffer adjusted to pH 10.0. The fibrinolytic proteins were eluted with the same buffer adjusted to pH 11.5. The column was run at a flow rate of 1.0 ml/min during application of the sample(s) and at 2.0 ml/min during the wash and elution steps. The effluent stream was monitored continuously at 280 nm, and fractions of 2 ml were collected throughout the chromatographic procedure. The column was regenerated by re-equilibration in the starting buffer defined above. Proteins thus treated were routinely 95% pure.

Purification of partially purified fibrinolytic proteins by reverse-phase HPLC

Fibrinolytic proteins purified by immunoaffinity chromatography or by alternate methods (e.g., lysine-Sepharose, SP-Sephadex, etc.) were routinely purified to homogeneity by reverse-phase HPLC employing a Varian LC5000 chromatographic system and a Vydac $C_4$ (0.46 x 26 cm) HPLC column. Samples were applied in either single or multiple injections (of various volumes up to 1 ml/injection) to the column, which had been equilibrated in a water solvent containing trifluoroacetic acid (0.1%, w/v). The column was developed with a linear gradient of increasing acetonitrile concentration (from 0% to 100% over 60 minutes) in the same TFA-containing solvent. The flow rate was constant at 1.0 ml/min, and fractions of 1 ml were collected. The effluent stream was monitored for absorbance at 215 nm, with a range of sensitivity depending on the predicted or known amount of starting material. Proteins purified by HPLC were used for amino acid sequence or compositional analyses.

Amino acid analysis

Amino acid analyses were performed in order to characterize amino acid compositions of HPLC-purified proteins and/or to obtain definitive data as to concentration of >95%-pure proteins in pools, stock solutions, etc. Pre-column derivatization of amino acid hydrolysates with phenylisothiocyanate (PITC) and reverse-phase HPLC was employed as a means to quantitative detection of phenylthiocarbamyl- (PTC-) amino acids. The procedure routinely employed was based on that of Heinrikson and Meredith (Anal. Biochem. 136: 65, 1984) as modified by Heinrikson, Mora and Maraganore (Modern Methods in Protein Chemistry, Pergamon Press, New York, 1987).

Sequence analysis

The automated Edman degradation was performed on single-chain or reduced, S-alkylated two-chain fragments that had been purified by reverse-phase HPLC. The purposes of these analyses were (1) to determine the extent of processing in mature single-chain t-PA homologs; (2) to verify the $NH_2$-terminal sequences in the various domain-deleted t-PA homologs; and (3) to determine the site of two-chain conversion in plasmin- or thrombin-activated fibrinolytic proteins. The chemical degradation was performed using an Applied Biosystems, Inc., 470A gas-phase sequencer. Phenylthiohydantoin- (PTH-) amino acids were separated and quantitated by reverse-phase HPLC employing either a Dupont PTH octadecylsilyl column and an isocratic gradient system developed by Dr. L. Erickson, University of Washington, Seattle, Washington, or an Applied Biosystems, Inc., 120A PTH-analyzer.

Plasma clearance of t-PA homologs

Plasmid clearance of authentic t-PA and t-PA homologs was tested in rats. Male Sprague Dawley rats (230 g to 270 g body weight) were injected with 0.4 mg/kg body weight of t-PA homologs or authentic recombinant t-PA purified from transfected BHK cells. Injection was via the femoral vein. Blood samples (0.5 ml) were withdrawn from the jugular vein and assayed for t-PA protein by a sandwich ELISA using affinity-purified polyclonal rabbit antibody. Results, shown in Table 7, indicate that several of the homologs have a plasma half-life of three to four times that of authentic t-PA.

TABLE 7

Plasma Half-Life of t-PA Homologs

| Protein | α Phase (minutes) | β Phase (minutes) |
|---|---|---|
| Authentic t-PA | 1.60 | 31.7 |
| 2625 | 5.10 | 83.2 |
| 2600 | 6.03 | 207.14 |
| 2700 | 7.10 | 71.62 |
| 2550 | 2.35 | 34.06 |
| 2725 | 11.91 | 39.23 |
| 1601 | 6.70 | – |
| 1606 | 2.47 | 33.37 |
| pMH10 | 2.38 | 73.52 |
| pMH13 | 2.70 | 48.34 |
| 820 | 1.25 | 38.17 |

In an alternate method, metabolically labeled mutant proteins were purified and assayed for in vivo half-life. Subconfluent 75 cm² flasks containing cells producing t-PA homologs or authentic t-PA (control) were washed in 10 ml methionine-free Dulbecco's MEM containing 100 U/ml penicillin, 100 μg/ml streptomycin, and 100 μg/ml aprotinin. The cells were incubated in 10 ml of the same medium containing 1 mci $^{35}$S-methionine (New England Nuclear) for 20 hours at 37°C. The supernatant was centrifuged at 2000 rpm for 10 minutes and stored at -20°C. Non-radioactive t-PAs were produced from cell layers in 1200 cm² trays (NUNC) maintained in Dulbecco's containing 100 U/ml penicillin, 100 μg/ml streptomycin, and 100 μg/ml aprotinin. Approximately 400 ml medium was harvested. NaCl and Tween-80 were added to the combined cell culture media to concentrations of 1 M and 0.2%, respectively. The pH was adjusted to 7.5. After filtration on a 0.45 μm filter, the proteins were purified using immunosorbent chromatography on monoclonal antibodies.

To determine in vivo half-life, female Wistar rats were anaesthetized and catheters were placed in the jugular vein for intravenous administration, and in the carotid artery for sampling. Heparin (1 mg/kg body weight) was given intravenously 10 minutes before administration of the test solution. Blood samples of approximately 20 μl were taken before and 2, 3, 4, 6 and 8 minutes after the administration of 0.25 to 0.5 ml test solutions. Radioactivity in plasma was determined by liquid scintillation. Elimination half-lives were calculated by linear regression using a one-compartment model. Data are presented in Table 8.

TABLE 8

| Protein | t½ (minutes) | Vd (ml/kg) |
|---|---|---|
| native t-PA | 2.3 ± 0.1 | 127 ± 4 |
| pMH10 | 2.1 ± 0.2 | 119 ± 27 |
| pMH13 | 2.0 (1.9-2.0) | 96 (90-101) |
| 3008 | 12 ± 1 | 44 ± 2 |
| 2600 | 18 ± 3 | 27 ± 2 |
| 820 | 10 (10-10) | 37 (36-38) |

Activity of t-PA homologs

Cell culture media were sterile filtered, and t-PA homologs were purified from the filtered media by affinity chromatography on Sepharose coupled to anti-t-PA monoclonal antibodies. The t-PA homologs were eluted from the column and assayed for activity using a variety of assays.

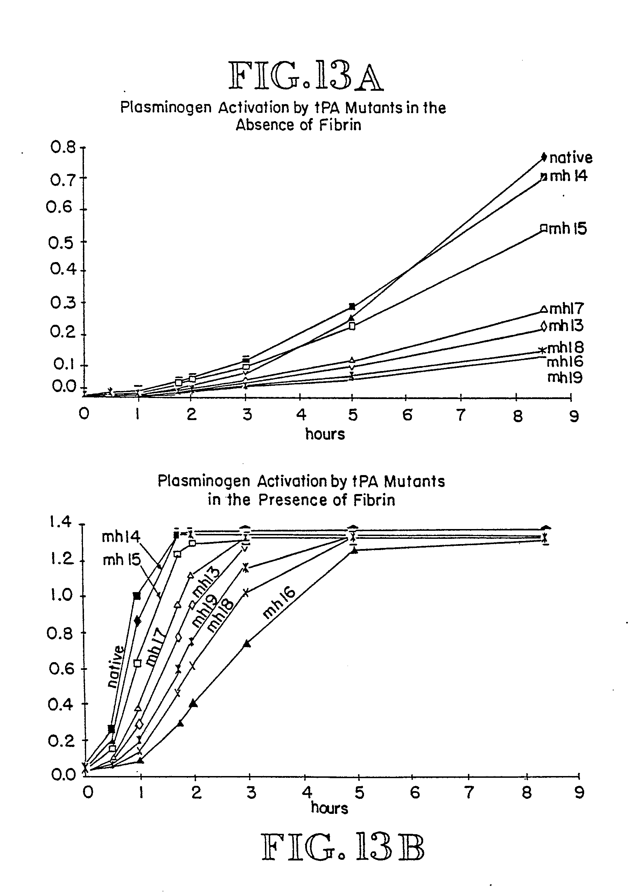

In comparison to native t-PA, the Gly (275) t-PA (pMH13) is 100 to 1000 times less sensitive to cleavage by plasmin, and is much more specific for activation in the presence of fibrin. It shows minimal activity in an amidolytic assay (Ichinose et al., FEBS Lett. 175: 412-418, 1984) (no fibrin present), even in the presence of large amounts of plasmin. In this assay, the amidolytic activity is due to the two-chain form of the molecule. In contrast, the native protein displays amidolytic activity in the presence of very small amounts of plasmin. In a fibrin-dependent activity assay (van Zonneveld et al., Proc. Natl. Acad. Sci. USA 83: 4670-4674, 1986), the Gly (275) mutant protein is resistant to activation and has little plasminogen-activating activity in the absence of fibrin, whereas native t-PA shows significant plasminogen-activating activity in the absence of fibrin. The two proteins exhibit comparable activity in the presence of fibrin (Figure 13). In a clot lysis assay, release of $^{125}$I-fibrin fragments is comparable for the native and mutant proteins. The pMH20 protein showed essentially no activity in the absence of fibrin, but in the presence of fibrin, showed activity similar to that of pMH13.

The pMH17 protein was designed to prevent cleavage of the Arg 275-Ile 276 bond by replacement of Ile (276) with Pro. However, the pMH17 protein was found to be completely converted by plasmin to a two-chain form. Surprisingly, no increase in amidolytic activity toward the substrate S-2444 was observed, suggesting that cleavage was occurring at a site which produced an inactive two chain form.

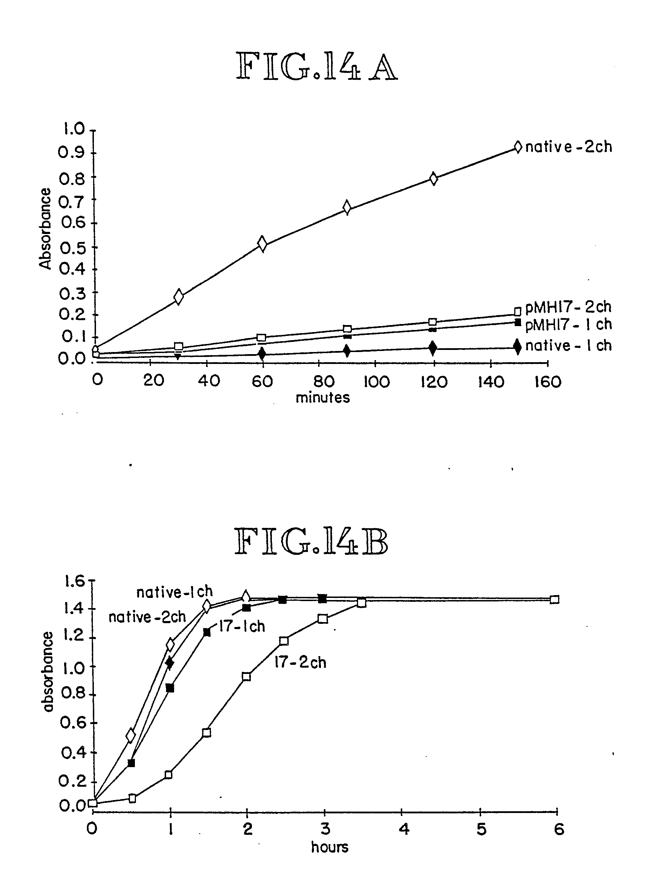

In order to investigate this possibility, a sample of pMH17 protein was completely converted to two-chain form by digestion with plasmin, and the conversion was verified by Western blotting. Then, samples of the single-chain form of pMH17 and also of the converted two-chain form were incubated with S-2444. Samples of single-chain and two-chain native t-PA were also assayed as controls. The results are shown in Figure 14a and indicate that, for native t-PA, the two-chain form shows considerably more amidolytic activity than the single-chain form in the absence of fibrin. The single-chain and two-chain forms of pMH17, however, showed identical- and low - amidolytic activity, confirming that there is no increase in this type of activity accompanying the conversion to two-chain form.

These two forms of pMH17 were then assayed for activity in conversion of plasminogen to plasmin in the presence and absence of fibrinogen fragments. The results (Figure 14b) indicate that, in the presence of fibrin, both single-chain and two-chain native t-PA cause equivalent conversion, whereas the single-chain form of pMH17 is considerably more active than the two-chain form. In the absence of fibrin, the two-chain form of native t-PA is considerably more active than the single-chain form, whereas, again, the single-chain form of pMH17 is more active than the two-chain form. These data support the idea that conversion to the two-chain form causes catalytic inactivation of pMH17, but that, in the presence of fibrin, even this form can be induced to show some plasminogen activation activity.

Pro (274) t-PA (pMH10) was assayed for activation by thrombin. One μg of t-PA was combined with 60 ng of bovine thrombin in 100 μl of TBS (50 mM Tris pH 7.5, 150 mM NaCl) + 0.1% Tween-80. The mixture was incubated at 37° C, and 25 μl aliquots were periodically removed and assayed for amidolytic activity by addition of 10 μl of 0.5 mM S-2444 in TBS + 0.1% Tween-80. After 60 minutes' incubation, the absorbance of the mixture at 405 nm was read. Results showed that the pMH10 protein was initially inactive but was activated by thrombin. Analysis by Western blot showed that this activation correlated with cleavage of the single-chain molecule to the two-chain form. In contrast, native t-PA showed neither activation nor cleavage by thrombin.

From the foregoing, it will be appreciated that, although specific embodiments of the invention have been described herein for purposes of illustration, various modifications may be made without deviating from the spirit and scope of the invention. Accordingly, the invention is not limited except as by the appended claims.

The features disclosed in the foregoing description, in the claims and/or in the accompanying drawings may, both separately and in any combination thereof, be material for realising the invention in diverse forms thereof.

## Claims

1. A t-PA homolog having the fibrinolytic capacity of native t-PA in the presence of fibrin, consisting essentially of an amino-terminal fibrin-binding domain and a carboxyl-terminal serine protease domain, wherein said homolog is essentially fibrin-binding inactive in the absence of fibrin, has greater resistance to cleavage by plasmin than native t-PA, and has increased plasma half-life as compared to native t-PA.

2. A t-PA homolog having the fibrinolytic capacity of native t-PA in the presence of fibrin, consisting essentially of an amino-terminal fibrin-binding domain and a carboxyl-terminal serine protease domain, said homolog having at least one amino acid substitution within four amino acid residues of the activation site and wherein said homolog is essentially fibrin-binding inactive in the absence of fibrin, has greater resistance to cleavage by plasmin than native t-PA, and has increased plasma half-life as compared to native t-PA.

3. The t-PA homolog of claims 1 or 2 wherein the amino- terminal fibrin-binding domain comprises at least one kringle structure.

4. The t-PA homolog of claim 3 wherein the amino-terminal fibrin-binding domain comprises two kringle structures.

5. The t-PA homolog of claim 4 wherein at least one of said kringle structures lacks carbohydrate.

6. The t-PA homolog of claim 5 wherein the amino-terminal fibrin-binding domain comprises essentially the K1 and K2 kringle structures of native t-PA and the K1 kringle structure is modified so as to lack carbohydrate.

7. The t-PA homolog of claims 1 or 2 wherein the amino- terminal fibrin-binding domain comprises at least one finger domain.

8. The t-PA homolog of claim 7 wherein the sequence of the finger domain is selected from the group consisting of those sequences set forth in Figure 6.

9. The t-PA homolog of claims 1 or 2 wherein the carboxyl- terminal serine protease domain is essentially the serine protease domain of t-PA.

10. The t-PA homolog of claims 1 or 2 wherein said t-PA homolog has an amino acid selected from the group consisting of Pro, Glu, Gly and Arg at the position corresponding to amino acid 274 of native t-PA.

11. The t-PA homolog of claims 1 or 2 wherein said t-PA homolog has an amino acid selected from the group consisting of Gly, Leu, Asp and Pro at the position corresponding to amino acid 275 of native t-PA.

12. The t-PA homolog of claims 1 or 2 wherein said t-PA homolog contains Leu at the position corresponding to amino acid 277 of native t-PA.

13. The t-PA homolog of claims 1 or 2 wherein said t-PA homolog contains Pro at the position corresponding to amino acid 276 of native t-PA.

14. The t-PA homolog of claims 1 or 2 wherein said t-PA homolog is activated by cleavage by thrombin.

15. The t-PA homolog of claim 2 having at least one amino acid substitution at an amino acid selected from the group consisting of amino acids corresponding to amino acids number 274, 275, 276 and 277 of native t-PA.

16. The t-PA homolog of claim 2 wherein said t-PA homolog contains a Leu at the position corresponding to amino acid 277 of native t-PA and contains Pro at the position corresponding to amino acid 274 of native t-PA.

17. The t-PA homolog of claim 2 wherein said t-PA homolog containing a Leu at the position corresponding to amino acid 277 of native t-PA and contains a Leu at the position corresponding to amino acid 275 of native t-PA.

18. A DNA construct encoding a t-PA homolog according to any of claims 1-17.

19. An expression vector capable of directing the expression of a t-PA homolog having the fibrinolytic capacity of native t-PA in the presence of fibrin, said vector including a promoter, said promoter being operably linked to a nucleotide sequence, said nucleotide sequence encoding said homolog and consisting essentially of a first region, encoding a fibrin-binding domain, and a second region positioned downstream of said first region, said second region encoding a catalytic domain for serine protease activity of native t-PA, wherein said t-PA homolog has at least one amino acid substitution at an amino acid selected from the group consisting of amino acids corresponding to amino acids numbers 274, 275, 276 and 277 of native t-PA.

20. A pharmaceutical composition comprising a t-PA homolog according to any of claims 1-17, in combination with a pharmaceutically acceptable carrier or diluent.

21. Cells transfected or transformed with the expression vector of claim 19.

22. Cells transfected or transformed to produce a protein according to any of claims 1-17.

23. A t-PA homolog according to any of claims 1-17, for use as an active therapeutic substance.

# FIG. 1

```
          10                    30                          45
AAGCTTGGAT CCACC ATG GAT GCA ATG AAG AGA GGG CTC TGC TGT GTG CTG CTG CTG
                  MET Asp Ala MET Lys Arg Gly Leu Cys Cys Val Leu Leu Leu
                  -35                           -30

  60                    75                      90                      105
TGT GGC GCC GTC TTC GTT TCG CCC AGC CAG GAA ATC CAT GCC CGA TTC AGA AGA
Cys Gly Ala Val Phe Val Ser Pro Ser Gln Glu Ile His Ala Arg Phe Arg Arg
    -20                                             -10

          120                     135                 150                 165
GGA GCC AGA TCT TAC CAA GTC ATC TGC AGA GAT GAA AAA ACG CAG ATG ATA TAC
Gly Ala Arg Ser Tyr Gln Val Ile Cys Arg Asp Glu Lys Thr Gln MET Ile Tyr
              1                                       10

CAG CAA CAT CAG TCA TGG CTG CGC CCT GTG CTC AGA AGC AAC CGC CTG GAA TAT
Gln Gln His Gln Ser Trp Leu Arg Pro Val Leu Arg Ser Asn Arg Val Glu Tyr
                  180                     195                 210
                      20                                      30

          225                     240                 255                 270
TGC TGG TGC AAC AGT GGC AGG GCA CAG TGC CAC TCA GTC CCT GTC AAA AGT TGC
Cys Trp Cys Asn Ser Gly Arg Ala Gln Cys His Ser Val Pro Val Lys Ser Cys
                      40                                          50

          285                     300               315
AGC CAG CCA AGG TGT TTC AAC GGG GGC ACC TGC CAG CAG GCC CTG TAC TTC TCA
Ser Gln Pro Arg Cys Phe Asn Gly Gly Thr Cys Gln Gln Ala Leu Tyr Phe Ser
                              60

330                     345                 360                 375
GAT TTC GTG TGC CAG TGC CCC GAA GGA TTT GCT GGG AAG TGC TGT GAA ATA GAT
Asp Phe Val Cys Gln Cys Pro Glu Gly Phe Ala Gly Lys Cys Cys Glu Ile Asp
 70                                             80

          390                     405                 420                 435
ACC AGG GCC AGC TGC TAC GAG GAC CAG GGC ATC AGC TAC AGG GGC ACG TGG AGC
Thr Arg Ala Ser Cys Tyr Glu Asp Gln Gly Ile Ser Tyr Arg Gly Thr Trp Ser
              90                                      100

          450                     465                 480
ACA GCG GAG AGT GGC GCC GAG TGC ACC AAC TGG AAC AGC AGC GCG TTG GCC CAG
Thr Ala Glu Ser Gly Ala Glu Cys Thr Asn Trp Asn Ser Ser Ala Leu Ala Gln
                  110                                 120

  495                     510                 525                 540
AAG CCC TAC AGC GGG CGG AGG CCA CAC GCC ATC AGG CTG GGC CTG CGG AAC CAC
Lys Pro Tyr Ser Gly Arg Arg Pro Asp Ala Ile Arg Leu Gly Leu Gly Asn His
                  130                                         140

          555                     570                 585
AAC TAC TGC AGA AAC CCA GAT CGA GAC TCA AAG CCC TGG TGC TAC GTC TTT AAG
Asn Tyr Cys Arg Asn Pro Asp Arg Asp Ser Lys Pro Trp Cys Tyr Val Phe Lys
                  150

600                     615                 630                 645
GCG GGG AAG TAC AGC TCA GAG TTC TGC AGC ACC CCT GCC TGC TCT CAG GGA AAC
Ala Gly Lys Tyr Ser Ser Glu Phe Cys Ser Thr Pro Ala Cys Ser Glu Gly Asn
160                                             170

          660                     675                 690                 705
AGT GAC TGC TAC TTT GGG AAT GGG TCA GCC TAC CGT GGC ACG CAC AGC CTC ACC
Ser Asp Cys Tyr Phe Gly Asn Gly Ser Ala Tyr Arg Gly Thr His Ser Leu Thr
              180                                     190
```

# FIG. 1 CONT.

```
                720                      735                      750
GAG TCG CGT GCC TCC TGC CTC CCG TGG AAT TCC ATG ATC CTG ATA GCC AAG GTT
Glu Ser Gly Ala Ser Cys Leu Pro Trp Asn Ser MET Ile Leu Ile Gly Lys Val
                200                                               210
      765                      780                      795                810
TAC ACA GCA CAG AAC CCC AGT GCC CAG GCA CTG GGC CTG GGC AAA CAT AAT TAC
Tyr Thr Ala Gln Asn Pro Ser Ala Gln Ala Leu Gly Leu Gly Lys His Asn Tyr
                               220                                    230
            825                      840                      855
TGC CGG AAT CCT CAT GGG GAT GCC AAG CCC TGG TGC CAC GTG CTG AAG AAC CGC
Cys Arg Asn Pro Asp Gly Asp Ala Lys Pro Trp Cys His Val Leu Lys Asn Arg
                                    240
870                      885                      900                      915
AGG CTG ACG TGG GAG TAC TGT GAT GTC CCC TCC TGC TCC ACC TGC GGC CTG AGA
Arg Leu Thr Trp Glu Tyr Cys Asp Val Pro Ser Cys Ser Thr Cys Gly Leu Arg
250                                               260
            930                      945                      960                975
CAG TAC AGC CAG CCT CAG TTT CGC ATC AAA GGA GGG CTC TTC GCC GAC ATC GCC
Gln Tyr Ser Gln Pro Gln Phe Arg Ile Lys Gly Gly Leu Phe Ala Asp Ile Ala
                270                                    280
            990                      1005                     1020
TCC CAC CCC TGG CAG GCT GCC ATC TTT GCC AAG CAC AGG AGG TCG CCC GGA GAG
Ser His Pro Trp Gln Ala Ala Ile Phe Ala Lys His Arg Arg Ser Pro Gly Glu
                290                                    300
      1035                     1050                     1065                1080
CGG TTC CTG TGC GGC GGC ATA CTC ATC AGC TCC TGC TGG ATT CTC TCT GCC CCC
Arg Phe Leu Cys Gly Gly Ile Leu Ile Ser Ser Cys Trp Ile Leu Ser Ala Ala
                               310                                    320
            1095                     1110                     1125
CAC TGC TTC CAG GAG AGG TTT CCG CCC CAC CAC CTG ACG GTG ATC TTG GGC AGA
His Cys Phe Gln Glu Arg Phe Pro Pro His His Leu Thr Val Ile Leu Gly Arg
                                    330
1140                     1155                     1170                     1185
ACA TAC CGG GTG GTC CCT GGC GAG GAG GAG CAG AAA TTT GAA GTC GAA AAA TAC
Thr Tyr Arg Val Val Pro Gly Glu Glu Glu Gln Lys Phe Glu Val Glu Lys Tyr
340                                               350
            1200                     1215                     1230                1245
ATT GTC CAT AAG GAA TTC GAT GAT·GAC ACT TAC GAC AAT GAC ATT GCG CTG CTG
Ile Val His Lys Glu Phe Asp Asp Asp Thr Tyr Asp Asn Asp Ile Ala Leu Leu
                360                                    370
            1260                     1275                     1290
CAG CTG AAA TCG GAT TCG TCC CGC TGT GCC CAG GAG AGC AGC GTG GTC CGC ACT
Gln Leu Lys Ser Asp Ser Ser Arg Cys Ala Gln Glu Ser Ser Val Val Arg Thr
                380                                               390
      1305                     1320                     1335                1350
GTG TGC CTT CCC CCG GCG GAC CTG CAG CTG CCG GAC TGG ACG GAG TGT GAG CTC
Val Cys Leu Pro Pro Ala Asp Leu Gln Leu Pro Asp Trp Thr Glu Cys Glu Leu
                               400                                    410
            ·1365                    1380                     1395
TCC GGC TAC GGC AAG CAT GAG GCC TTG TCT CCT TTC TAT TCG GAG CGG CTC AAG
Ser Gly Tyr Gly Lys His Glu Ala Leu Ser Pro Phe Tyr Ser Glu Arg Leu Lys
                               420
```

```
1410              1425                 1440                 1425
GAG GCT CAT GTC AGA CTG TAC CCA TCC AGC CCC TGC ACA TCA CAA CAT TTA CTT
Glu Ala His Val Arg Leu Tyr Pro Ser Ser Arg Cys Thr Ser Gln His Leu Leu
430                                  440
         1470                 1485                 1500                 1515
AAC AGA ACA GTC ACC GAC AAC ATG CTG TGT GCT GGA GAC ACT CGG AGC GGC GGG
Asn Arg Thr Val Thr Asp Asn MET Leu Cys Ala Gly Asp Thr Arg Ser Gly Gly
         450                                  460
              1530                 1545                 1560
CCC CAG GCA AAC TTG CAC GAC GCC TGC CAG GCC CAT TCG GGA GGC CCC CTG GTG
Pro Gln Ala Asn Leu His Asp Ala Cys Gln Gly Asp Ser Gly Gly Pro Leu Val
                   470                                  480
         1575                 1590                 1605                 1620
TGT CTG AAC GAT GGC CGC ATG .CT TTG GTG GGC ATC ATC AGC TGG GGC CTG GGC
Cys Leu Asn Asp Gly Arg MET Thr Leu Val Gly Ile Ile Ser Trp Gly Leu Gly
                        490                                  500
              1635                 1650                 1665
TGT GGA CAG AAG GAT GTC CCG GGT GTG TAC ACA AAG GTT ACC AAC TAC CTA GAC
Cys Gly Gln Lys Asp Val Pro Gly Val Tyr Thr Lys Val Thr Asn Tyr Leu Asp
                             510
1680              1695                 1714      1724      1734
TGG ATT CGT GAC AAC ATG CGA CCG TGA CCAGGAACAC CCGACTCCTC AAAAGCAAAT GAGATC
Trp Ile Arg Asp Asn MET Arg Pro
520                   527
```

# FIG. 1 CONT.

FIG. 2

EP 0 292 009 A1

# FIG.3

## FIG. 4

A. PRE-PRO-SER-CYS-THR-ASN-TRP- [1 113]

B. PRE-PRO-SER-TYR-GLN-VAL-THR-ARG-ALA- [1 4 88]

C. PRE-PRO-SER-TYR-GLN-VAL-GLY-ASN-SER-ASP- [1 4 176]

D. PRE-PRO-SER-TYR-GLN-VAL-ILE-CYS-ARG-ASP-GLU-LYS-THR-GLN-MET- [1 10]

ILE-TYR-GLN-GLN-HIS-GLN-SER-TRP-LEU-ARG-PRO-VAL-LEU- [20]

ARG-SER-ASN-ARG-VAL-GLU-TYR-CYS-TRP-CYS-ASN-SER-GLY- [30]

ARG-ALA-GLN-CYS-HIS-SER-VAL-PRO-VAL-LYS-THR-ARG-ALA- [40 49 88]

EP 0 292 009 A1

# FIG.5

6A   CRDEKTQMIYQQHQSWLRPVLRSNRVEYCWCNSGRAQC

6B   CFDNGKSYKIGETWERPYEGFMLSCTCLGNGRGEFRC

6C   CHDEKTGSSYKIGEQWERPYLSGNRLECTCLGNGSGRWQC

6D   CFDNGKSYKIGETWERPYEGFMLSCTCLGNGSGRWQC

6E   CFDNGKSYKIGEQWERPYLSGNRLECTCLGNGRGEFRC

6F   CFDNGKSYKIGEQWERPYLSGNRLECTCLGNGSGRWQC

6G   CHDEKTGSSYKIGETWERPYEGFMLSCTCLGNGSGRWQC

6H   CHDEKTGSSYKIGEQWERPYLSGNRLECTCLGNGRGEFRC

6I   CHDEKTGSSYKIGETWERPYEGFMLSCTCLGNGRGEFRC

# FIG.6

# FIG. 7

```
*   1                                                                    80*
ATG.GAT.GCA.ATG.AAG.AGA.GGG.CTC.TGC.TGT.GTG.CTG.CTG.CTG.TGT.GGC.GCC.GTC.TTC.GTT
MET-Asp-Ala-MET-Lys-Arg-Gly-Leu-Cys-Cys-Val-Leu-Leu-Leu-Cys-Gly-Ala-Val-Phe-Val

*   81                                                                   120*
TCG.CCC.AGC.CAG.GAA.ATC.CAT.GCC.CGA.TTC.AGA.AGA.GGA.GCC.AGA.TCT.TAC.CAA.GAT.ACC
Ser-Pro-Ser-Gln-Glu-Ile-His-Ala-Arg-Phe-Arg-Arg-Gly-Ala-Arg-Ser-Tyr-Gln-Asp-Thr

*   121                                                                  180*
AGG.GCC.ACG.TGC.TAC.GAG.GAC.CAG.GGC.ATC.AGC.TAC.AGG.GGC.ACG.TGG.AGC.ACA.GCG.GAG
Arg-Ala-Thr-Cys-Thr-Glu-Asp-Gln-Gly-Ile-Ser-Tyr-Arg-Gly-Thr-Trp-Ser-Thr-Ala-Glu

*   181                                                                  240*
AGT.GGC.GCC.GAG.TGC.ACC.AAC.TGG.AAC.AGC.AGC.GCG.TTG.GCC.CAG.AAG.CCC.TAC.AGC.GGG
Ser-Gly-Ala-Glu-Cys-Thr-Asn-Trp-Asn-Ser-Ser-Ala-Leu-Ala-Gln-Lys-Pro-Tyr-Ser-Gly

*   241                                                                  300*
CGG.AGG.CCA.GAC.GCC.ATC.AGG.CTG.GGC.CTG.GGG.AAC.CAC.AAC.TAC.TGC.AGA.AAC.CCA.GAT
Arg-Arg-Pro-Asp-Ala-Ile-Arg-Leu-Gly-Leu-Gly-Asn-His-Asn-Tyr-Cys-Arg-Asn-Pro-Asp

*   301                                                                  360*
CGA.GAC.TCA.AAG.CCC.TGG.TGC.TAC.GTC.TTT.AAG.GCG.GGG.AAG.TAC.AGC.TCA.GAG.TTC.TGC
Arg-Asp-Ser-Lys-Pro-Trp-Cys-Tyr-Val-Phe-Lys-Ala-Gly-Lys-Tyr-Ser-Ser-Glu-Phe-Cys

*   361                                                                  420*
AGC.ACC.CCT.GCC.TGC.TCT.GAG.GGA.AAC.AGT.GAC.TGC.TAC.TTT.GGG.AAT.GGG.TCA.GCC.TAC
Ser-Thr-Pro-Ala-Cys-Ser-Glu-Gly-Asn-Ser-Asp-Cys-Tyr-Phe-Gly-Asn-Gly-Ser-Ala-Tyr

*   421                                                                  480*
CGT.GGC.ACG.CAC.AGC.CTC.ACC.GAG.TCG.GGT.GCC.TCC.TGC.CTC.CCG.TGG.AAT.TCC.ATG.ATC
Arg-Gly-Thr-His-Ser-Leu-Thr-Glu-Ser-Gly-Ala-Ser-Cys-Leu-Pro-Trp-Asn-Ser-MET-Ile

*   481                                                                  540*
CTG.ATA.GGC.AAG.GTT.TAC.ACA.GCA.CAG.AAC.CCC.AGT.GCC.CAG.GCA.CTG.GGC.CTG.GGC.AAA
Leu-Ile-Gly-Lys-Val-Tyr-Thr-Ala-Gln-Asn-Pro-Ser-Ala-Gln-Ala-Leu-Gly-Leu-Gly-Lys

*   541                                                                  600*
CAT.AAT.TAC.TGC.CGG.AAT.CCT.GAT.GGG.GAT.GCC.AAG.CCC.TGG.TGC.CAC.GTG.CTG.AAG.AAC
His-Asn-Tyr-Cys-Arg-Asn-Pro-Asp-Gly-Asp-Ala-Lys-Pro-Trp-Cys-His-Val-Leu-Lys-Asn

*   601                                                                  660*
CGC.AGG.CTG.ACG.TGG.GAG.TAC.TGT.GAT.GTG.CCC.TCC.TGC.TCC.ACC.TGC.GGC.CTG.AGA.CAG
Arg-Arg-Leu-Thr-Trp-Glu-Tyr-Cys-Asp-Val-Pro-Ser-Cys-Ser-Thr-Cys-Gly-Leu-Arg-Gln

*   661                                                                  720*
TAC.AGC.CAG.CCT.CAG.TTT.CGC.ATC.AAA.GGA.GGG.CTC.TTC.GCC.GAC.ATC.GCC.TCC.CAC.CCC
Tyr-Ser-Gln-Pro-Gln-Phe-Arg-Ile-Lys-Gly-Gly-Leu-Phe-Ala-Asp-Ile-Ala-Ser-His-Pro

*   721                                                                  780*
TGG.CAG.GCT.GCC.ATC.TTT.GCC.AAG.CAC.AGG.AGG.TCG.CCC.GGA.GAG.CGG.TTC.CTG.TGC.GGG
Trp-Gln-Ala-Ala-Ile-Phe-Ala-Lys-His-Arg-Arg-Ser-Pro-Gly-Glu-Arg-Phe-Leu-Cys-Gly
```

```
*  781                                                                    840*
GGC.ATA.CTC.ATC.AOC.TCC.TGC.TGG.ATT.CTC.TCT.GCC.GCC.CAC.TGC.TTC.CAG.GAG.AGG.T.T
Gly-Ile-Leu-Ile-Ser-Ser-Cys-Trp-Ile-Leu-Ser-Ala-Ala-His-Cys-Phe-Gln-Glu-Arg-Phe

*  841                                                                    900*
CCG.CCC.CAC.CAC.CTG.ACG.GTG.ATC.TTG.GGC.AGA.ACA.TAC.CGG.GTG.GTC.CCT.GGC.GAG.GAG
Pro-Pro-His-His-Leu-Thr-Val-Ile-Leu-Gly-Arg-Thr-Tyr-Arg-Val-Val-Pro-Gly-Glu-Glu

*  901                                                                    960*
GAG.CAG.AAA.TTT.GAA.GTC.GAA.AAA.TAC.ATT.GTC.CAT.AAG.GAA.TTC.GAT.GAT.GAC.ACT.TAC
Glu-Gln-Lys-Phe-Glu-Val-Glu-Lys-Tyr-Ile-Val-His-Lys-Glu-Phe-Asp-Asp-Asp-Thr-Tyr

*  961                                                                   1020*
GAC.AAT.GAC.ATT.GCG.CTG.CTG.CAG.CTG.AAA.TCG.GAT.TCG.TCC.CGC.TGT.GCC.CAG.GAG.AGC
Asp-Asn-Asp-Ile-Ala-Leu-Leu-Gln-Leu-Lys-Ser-A  n-Ser-Ser-Arg-Cys-Ala-Gln-Glu-Ser

* 1021                                                                   1080*
AGC.GTG.GTC.CGC.ACT.GTG.TGC.CTT.CCC.CCG.GCG.GAC.CTG.CAG.CTG.CCG.GAC.TGG.ACG.GAG
Ser-Val-Val-Arg-Thr-Val-Cys-Leu-Pro-Pro-Ala-Asp-Leu-Gln-Leu-Pro-Asp-Trp-Thr-Glu

* 1081                                                                   1140*
TGT.GAG.CTC.TCC.GGC.TAC.GGC.AAG.CAT.GAG.GCC.TTG.TCT.CCT.TTC.TCT.TCG.GAG.CGG.CTG
Cys-Glu-Leu-Ser-Gly-Tyr-Gly-Lys-His-Glu-Ala-Leu-Ser-Pro-Phe-Tyr-Ser-Glu-Arg-Leu

* 1141                                                                   1200*
AAG.GAG.GCT.CAT.GTC.AGA.CTG.TAC.CCA.TCC.AGC.CGC.TGC.ACA.TCA.CAA.CAT.TTA.CTT.AAC
Lys-Glu-Ala-His-Val-Arg-Leu-Tyr-Pro-Ser-Ser-Arg-Cys-Thr-Ser-Gln-His-Leu-Leu-Asn

* 1201                                                                   1280*
AGA.ACA.GTC.ACC.GAC.AAC.ATG.CTG.TGT.GCT.GGA.GAC.ACT.CGG.AGC.GGC.GGG.CCC.CAG.GCA
Arg-Thr-Val-Thr-Asp-Asn-HET-Leu-Cys-Ala-Gly-Asp-Thr-Arg-Ser-Gly-Gly-Pro-Gln-Ala

* 1261                                                                   1320*
AAC.TTG.CAC.GAC.GCC.TGC.CAG.GGC.GAT.TCG.GGA.GGC.CCC.CTG.GTG.TGT.CTG.AAC.GAT.GGC
Asn-Leu-His-Asp-Ala-Cys-Gln-Gly-Asp-Ser-Gly-Gly-Pro-Leu-Val-Cys-Leu-Asn-Asp-Gly

* 1321                                                                   1380*
CGC.ATG.ACT.TTG.GTG.GGC.ATC.ATC.AGC.TGG.GGC.CTG.GGC.TGT.GGA.CAG.AAG.GAT.GTC.CCG
Arg-HET-Thr-Leu-Val-Gly-Ile-Ile-Ser-Trp-Gly-Leu-Gly-Cys-Gly-Gln-Lys-Asp-Val-P..o

* 1381                                                                   1440*
GGT.GTG.TAC.ACA.AAG.GTT.ACC.AAC.TAC.CTA.GAC.TGG.ATT.CGT.GAC.AAC.ATG.CGA.CCG.TGA
Gly-Val-Tyr-Thr-Lys-Val-Thr-Asn-Tyr-Lau-Asp-Trp-Ile-Arg-Asp-Asn-HET-Arg-Pro-***
```

# FIG. 7 CONT.

## FIG.8

```
*    1                                                                         80*
ATG.GAT.GCA.ATG.AAG.AGA.GGG.CTC.TGC.TGT.GTG.CTG.CTG.CTG.TGT.GGC.GCC.GTC.TTC.GTT
MET-Asp-Ala-HET-Lys-Arg-Gly-Leu-Cys-Cys-Val-Leu-Leu-Leu-Cys-Gly-Ala-Val-Phe-Val

*    81                                                                        120*
TCG.CCC.AGC.CAG.GAA.ATC.CAT.GCC.CGA.TTC.AGA.AGA.GGA.GCC.AGA.TCT.TAC.CAA.GGA.AAC
Ser-Pro-Ser-Gln-Glu-Ile-His-Ala-Arg-Phe-Arg-Arg-Gly-Ala-Arg-Ser-Tyr-Gln-Gly-Asn

*    121                                                                       180*
AGT.GAC.TGC.TAC.TTT.GGG.AAT.GGG.TCA.GCC.TAC.CGT.GGC.ACG.CAC.AGC.CTC.ACC.GAG.TCG
Ser-Asp-Cys-Tyr-Phe-Gly-Asn-Gly-Ser-Ala-Tyr-Arg-Gly-Thr-His-Ser-Leu-Thr-Glu-Ser

*    181                                                                       240*
GGT.GCC.TCC.TGC.CTC.CCG.TGG.AAT.TCC.ATG.ATC.CTG.ATA.GGC.AAG.GTT.TAC.ACA.GCA.CAG
Gly-Ala-Ser-Cys-Leu-Pro-Trp-Asn-Ser-HET-Ile-Leu-Ile-Gly-Lys-Val-Ty -Thr-Ala-Gln

*    241                                                                       300*
AAC.CCC.AGT.GCC.CAG.GCA.CTG.GGC.CTG.GGC.AAA.CAT.AAT.TAC.TGC.CGG.AAT.CCT.GAT.GGG
Asn-Pro-Ser-Ala-Gln-Ala-Leu-Gly-Leu-Gly-Lys-His-Asn-Tyr-Cys-Arg-Asn-Pro-Asp-Gly

*    301                                                                       360*
GAT.GCC.AAG.CCC.TGG.TGC.CAC.GTG.CTG.AAG.AAC.CGC.AGG.CTG.ACG.TGG.GAG.TAC.TGT.GAT
Asp-Ala-Lys-Pro-Trp-Cys-His-Val-Leu-Lys-Asn-Arg-Arg-Leu-Thr-Trp-Glu-Tyr-Cys-Asp

*    361                                                                       420*
GTG.CCC.TCC.TGC.TCC.ACC.TGC.GGC.CTG.AGA.CAG.TAC.AGC.CAG.CCT.CAG.TTT.CGC.ATC.AAA
Val-Pro-Ser-Cys-Ser-Thr-Cys-Gly-Leu-Arg-Gln-Tyr-Ser-Oln-Pro-Oln-Phe-Arg-Ile-Lys

*    421                                                                       480*
GGA.GGG.CTC.TTC.GCC.GAC.ATC.GCC.TCC.CAC.CCC.TGG.CAG.GCT.GCC.ATC.TTT.GCC.AAG.CAC
Gly-Gly-Leu-Phe-Ala-Asp-Ile-Ala-Ser-His-Pro-Trp-Gln-Ala-Ala-Ile-Phe-Ala-Lys-His

*    481                                                                       540*
AGG.AGG.TCG.CCC.CGA.GAG.CGG.TTC.CTG.TGC.GGG.GGC.ATA.CTC.ATC.AGC.TCC.TGC.TGG.ATT
Arg-Arg-Ser-Pro-Gly-Glu-Arg-Phe-Leu-Cys-Gly-Gly-Ile-Leu-Ile-Ser-Ser-Cys-Trp-Ile

*    541                                                                       600*
CTC.TCT.GCC.GCC.CAC.TGC.TTC.CAG.GAG.AGG.TTT.CCG.CCC.CAC.CAC.CTG.ACG.GTG.ATC.TTG
Leu-Ser-Ala-Ala-His-Cys-Phe-Gln-Glu-Arg-Phe-Pro-Pro-His-His-Leu-Thr-Val-Ile-Leu

*    601                                                                       660*
GGC.AGA.ACA.TAC.CGG.GTG.GTC.CCT.GGC.GAG.GAG.GAG.CAG.AAA.TTT.GAA.GTC.GAA.AAA.TAC
Gly-Arg-Thr-Tyr-Arg-Val-Val-Pro-Gly-Gly-Glu-Glu-Gln-Lys-Phe-Olu-Val-Glu-Lys-Tyr

*    661                                                                       720*
ATT.GTC.CAT.AAG.GAA.TTC.GAT.GAT.GAC.ACT.TAC.GAC.AAT.GAC.ATT.GCG.CTG.CTG.CAG.CTG
Ile-Val-His-Lys-Glu-Phe-Asp-Asp-Asp-Thr-Tyr-Asp-Asn-Asp-Ile-Ala-Leu-Leu-Gln-Leu

*    721                                                                       780*
AAA.TCG.GAT.TCG.TCC.CGC.TGT.GCC.CAG.GAG.AGC.AGC.GTG.GTC.CGC.ACT.GTG.TGC.CTT.CCC
Lys-Ser-Asp-Ser-Ser-Arg-Cys-Ala-Gln-Glu-Ser-Ser-Val-Val-Arg-Thr-Val-Cys-Leu-Pro
```

```
*   781                                                              840*
CCG.GCG.GAC.CTG.CAG.CTG.CCG.GAC.TGG.ACG.GAG.TGT.GAG.CTC.TCC.GGC.TAC.GGC.AAG.CAT
Pro-Ala-Asp-Leu-Gln-Leu-Pro-Asp-Trp-Thr-Glu-Cys-Glu-Leu-Ser-Gly-Tyr-Gly-Lys-His

*   841                                                              900*
GAG.GCC.TTG.TCT.CCT.TTC.TAT.TCG.GAG.CGG.CTG.AAG.GAG.GCT.CAT.GTC.AGA.CTG.TAC.CCA
Glu-Ala-Leu-Ser-Pro-Phe-Tyr-Ser-Glu-Arg-Leu-Lys-Glu-Ala-His-Val-Arg-Leu-Tyr-Pro

*   901                                                              960*
TCC.AGC.CGC.TGC.ACA.TCA.CAA.CAT.TTA.CTT.AAC.AGA.ACA.GTC.ACC.CAC.AAC.ATG.CTG.TGT
Ser-Ser-Arg-Cys-Thr-Ser-Gln-His-Leu-Leu-Asn-Arg-Thr-Val-Thr-Asp-Asn-HET-Leu-Cys

*   961                                                             1020*
GCT.GGA.GAC.ACT.CGG.AGC.GGC.GGG.CCC.CAG.GCA.AAC.TTG.CAC.GAC.GCC.TGC.CAG.GGC.GAT
Ala-Gly-Asp-Thr-Arg-Ser-Gly-Gly-Pro-Gln-Ala-Asn-Leu-His-Asp-Ala-Cys-Gln-Gly-Asp

*  1021                                                             1080*
TCG.GGA.GGC.CCC.CTG.GTG.TGT.CTG.AAC.GAT.GGC.CGC.ATG.ACT.TTG.GTG.GGC.ATC.ATC.AGC
Ser-Gly-Gly-Pro-Leu-Val-Cys-Leu-Asn-Asp-Gly-Arg-HET-Thr-Leu-Val-Gly-Ile-Ile-Ser

*  1081                                                             1140*
TGG.GGC.CTG.GGC.TGT.GGA.CAG.AAG.GAT.GTC.CCC.GGT.GTG.TAC.ACA.AAG.GTT.ACC.AAC.TAC
Trp-Gly-Leu-Gly-Cys-Gly-Gln-Lys-Asp-Val-Pro-Gly-Val-Tyr-Thr-Lys-Val-Thr-Asn-Tyr

*  1141
CTA.GAC.TGG.ATT.CGT.GAC.AAC.ATG.CGA.CCG.TGA
Leu-Asp-Trp-Ile-Arg-Asp-Asn-HET-Arg-Pro-***
```

## FIG.8 CONT.

EP 0 292 009 A1

EcoRI    Eco RI
tPA
BglⅡ/BamHI              XhoⅡ/BglⅡ
HindⅢ    hGH
terminator    BamHI/BglⅡ
EcoRI
EcoRI
SV40
promoter
Zem 94

BglⅡ/BamHI
HindⅢ    5'tPA    EcoRI
MI3mpI9

BamHI
HindⅢ
5'tPA
MI3-modified
translation start
site
MI3mpI9

3'tPA-hGH EcoRI
fragment from Zem99
BamHI and EcoRI
cut pICI9H

EcoRI
BamHI
HindⅢ    5'tPA    3'tPA    XbaI
hGH
terminator
HindⅢ
Zem 182
pICI9H    EcoRI
HindⅢ

EcoRI
BamHI
HindⅢ    5'tPA    3'tPA
hGH
terminator    XbaI
HindⅢ
Zem 182b

FIG.9

FIG. 10

EP 0 292 009 A1

FIG. 11

# FIG.12

# FIG. 13A

Plasminogen Activation by tPA Mutants in the
Absence of Fibrin

hours

Plasminogen Activation by tPA Mutants
in the Presence of Fibrin

hours

# FIG. 13B

# FIG.14A

Absorbance vs minutes

- native-2ch
- pMHl7-2ch
- pMHl7-1ch
- native-1ch

# FIG.14B

absorbance vs hours

- native-1ch
- native-2ch
- 17-1ch
- 17-2ch

| | DOCUMENTS CONSIDERED TO BE RELEVANT | | EP 88108148.3 |
|---|---|---|---|

| Category | Citation of document with indication, where appropriate, of relevant passages | Relevant to claim | CLASSIFICATION OF THE APPLICATION (Int. Cl.4) |
|---|---|---|---|
| D,X | EP - A2 - 0 199 574 (GENENTECH, INC.)<br>  * Claims 1,5-9,12-14,19,20 *<br>& GB-A-2 173 804<br>-- | 1,2,11, 15,18-23 | C 12 N   9/50<br>C 12 N  15/00<br>C 07 H  21/04<br>A 61 K  37/54<br>C 12 N   5/00 |
| P,X | EP - A2 - 0 233 013 (BEECHAM GROUP PLC)<br>  * Claims 1-3,7-9,12,13 *<br>-- | 1,2,9, 11,15, 18-23 | |
| D,X | WO - A1 - 86/01 538 (BIOGEN N.V.)<br>  * Claims 1,3,10,12,27,28 *<br>-- | 1,2,12, 18,20, 22 | |
| P,X | EP - A2 - 0 227 462 (CHIRON COR-PORATION)<br>  * Claims 1,2,10,12,23-25,29 *<br>-- | 1,2,9, 15,18-23 | |
| | | | **TECHNICAL FIELDS SEARCHED (Int. Cl.4)** |
| D,X | WO - A1 - 84/01 786 (BEECHAM GROUP P.L.C.)<br>  * Abstract *<br>-- | 1,4-6 | C 12 N<br>C 07 H<br>A 61 K |
| P,A | EP - A1 - 0 241 210 (BEECHAM GROUP PLC)<br>  * Claims 1,14-16,20,22 *<br>-- | 1,7,18-23 | |

The present search report has been drawn up for all claims

| Place of search<br>VIENNA | Date of completion of the search<br>30-08-1988 | Examiner<br>WOLF |
|---|---|---|

European Patent Office

**EUROPEAN SEARCH REPORT**

Application number

| | DOCUMENTS CONSIDERED TO BE RELEVANT | | EP 88108148.3 |
|---|---|---|---|
| Category | Citation of document with indication, where appropriate, of relevant passages | Relevant to claim | CLASSIFICATION OF THE APPLICATION (Int. Cl.4) |
| D,A | THE JOURNAL OF BIOLOGICAL CHEMISTRY, vol. 261, no. 30, October 25, 1986 (Baltimore, USA) <br><br> A.-J. VAN ZONNEVELD et al. "On the Interaction of the Finger and the Kringle-2 Domain of Tissue-type Plasminogen Activator with Fibrin" pages 14214-14218 <br><br> * Abstract * <br><br> -- | 1,3-7 | |
| D,A | PROCEEDINGS OF THE NATIONAL ACADEMY OF SCIENCES OF THE UNITED STATES OF AMERICA, vol. 83, no. 13, July 1986, (Baltimore, USA) <br><br> A.-J. VAN ZONNEVELD et al. "Autonomous functions of structural domains on human tissue-type plasminogen activator" pages 4670-4674 <br><br> * Abstract * <br><br> -- | 1,3,4, 6,7 | TECHNICAL FIELDS SEARCHED (Int. Cl.4) |
| D,A | PROCEEDINGS OF THE NATIONAL ACADEMY OF SCIENCES OF THE UNITED STATES OF AMERICA, vol. 81, no. 17, September 1984 (Baltimore, USA) <br><br> T. NY et al. "The structure of the human tissue-type plasminogen activator gene: Correction of intron and exon structures to functional and structural domains" pages 5355-5359 <br><br> * Totality * | | |

The present search report has been drawn up for all claims

| Place of search | Date of completion of the search | Examiner |
|---|---|---|
| VIENNA | 30-08-1988 | WOLF |